# EUROPÄISCHE PATENTSCHRIFT

(11) **EP 3 212 694 B1**
(45) Veröffentlichungstag und Bekanntmachung des Hinweises auf die Patenterteilung: **27.03.2019**
(21) Anmeldenummer: 15786939.7
(22) Anmeldetag: 27.10.2015
(51) Int. Cl.: C08G 77/14, A61Q 11/00, A61K 6/00, C07F 7/18, C08G 77/28, C08L 83/06, C08L 83/08, C08G 77/00

(54) **MIT AROMATISCHEN RESTEN UND MIT HYDROXYGRUPPEN SUBSTITUIERTE SILANE UND KIESELSÄURE(HETERO)POLYKONDENSATE SOWIE DERIVATE DAVON, DIE SICH ALS SOLCHE ODER ALS KOMPOSITE FÜR (MATRIX-)SYSTEME MIT HOHER TRANSLUZENZ UND GUTER MECHANIK EIGNEN**
SILANES AND SILICIC ACID (HETERO) POLYCONDENSATES SUBSTITUTED WITH AROMATIC RESIDUES AND WITH HYDROXY GROUPS, AND DERIVATIVES THEREOF, WHICH ARE SUITABLE, AS SUCH OR AS COMPOSITES, FOR (MATRIX) SYSTEMS HAVING HIGH TRANSLUCENCE AND GOOD MECHANICAL PROPERTIES
SILANES ET (HÉTÉRO)POLYCONDENSATS D'ACIDE SILICIQUE SUBSTITUÉS PAR DES RESTES AROMATIQUES ET DES GROUPES HYDROXY AINSI QUE DÉRIVÉS DE CES DERNIERS, QUI CONVIENNENT EN TANT QUE TELS OU SOUS FORME COMPOSITE POUR DES SYSTÈMES (MATRICIELS) PRÉSENTANT UNE TRANSLUCIDITÉ ÉLEVÉE ET DE BONNES PROPRIÉTÉS MÉCANIQUES

(30) Priorität: 29.10.2014 DE 102014115751
(43) Veröffentlichungstag der Anmeldung: 06.09.2017
(73) Patentinhaber: Wolter, Herbert, 97941 Tauberbischofsheim (DE); Nique, Somchith, 97249 Eisingen (DE)
(72) Erfinder: WOLTER, Herbert, 97941 Tauberbischofsheim (DE); NIQUE, Somchith, 97249 Eisingen (DE)
(74) Vertreter: Strehl Schübel-Hopf & Partner
(86) Internationale Anmeldenummer: PCT/EP2015/074899
(87) Internationale Veröffentlichungsnummer: WO 2016/066653

(56) Entgegenhaltungen:
- WO-A1-2013/053745
- WO-A1-2015/018906

## Beschreibung

Die vorliegende Erfindung betrifft in ihrer ersten Ausgestaltung anorganisch-organische Materialien, nämlich Silane, Harze und Matrixsysteme (Kieselsäure(hetero)polykondensate) mit freien Hydroxygruppen, mit aromatischen Resten und fakultativ weiterhin mit reaktiven, cyclischen Ethergruppen. Aufgrund der Wahl des Herstellungsverfahrens kann dabei ausgehend von einem einzigen Silan- oder (Hetero)polysiloxan-Ausgangssystem trotz Anwendung nur eines Verfahrensschritts der (molare) Anteil der aromatischen Reste relativ zum (molaren) Anteil der Hydroxygruppen und zum (molaren) Anteil an cyclischen Ethergruppen völlig variabel eingestellt werden.

In einer zweiten Ausgestaltung der Erfindung können die Materialien anstelle von reaktiven cyclischen Ethergruppen oder sogar zusätzlich dazu polymerisationsfähige C=C-Doppelbindungen aufweisen, so dass sie organisch vernetzbar sind oder organisch vernetzt vorliegen. Diese Materialien lassen sich ausgehend von demselben Silan- oder (Hetero)polysiloxan-Ausgangssystem in maximal zwei, manchmal sogar nur in einem einzigen Verfahrensschritt erzeugen.

Überdies ist es möglich, die (molaren) Anteile der jeweiligen Gruppen relativ zur (molaren) Menge an Silicium stufenlos einzustellen, und zwar sowohl auf der Stufe der Silane selbst (indem durch den Einsatz von Reaktionspartnern im Unterschuss Gemische erzeugt werden) als auch auf der Stufe der Kieselsäure(hetero)polykondensate.

Das Vorhandensein der zwei, drei oder sogar vier verschiedenen genannten Gruppen in variablen Anteilen relativ zueinander und zum Silicium ermöglicht die Einstellung einer Vielzahl von mechanischen und weiteren physikalischen Parametern in den erfindungsgemäßen Materialien. Auf verschiedenen Gebieten lässt sich ein solches Material, das gegebenenfalls außerdem mit Füllstoffen befüllt werden kann, daher in besonders günstiger Weise einsetzen, Dazu gehört der Dentalbereich, bei dem mit der Einstellung der gewünschten Transluzenz eine speziell geforderte Ästhetik realisiert werden kann, während die Steuerungsmöglichkeit für die Strahlungsdurchlässigkeit einen Einsatz z.B. bei optischen Anwendungen ermöglicht, ohne dass die Erzielung der gewünschten jeweiligen mechanischen Eigenschaften, z.B. eines speziellen E-Moduls, dadurch nachteilig beeinträchtigt wäre. Ein besonderer Vorteil der Systeme liegt darin, dass sich Harze und Matrixsysteme erzeugen lassen, die sich nicht nur bezüglich der Brechzahl bzw. Transluzenz stufenlos bzw. sehr fein abgestuft einstellen lassen, sondern auch bezüglich der Bruchfestigkeit, des E-Moduls und der Biegbarkeit.

Im Bereich der dentalen Werksstoffe, aber nicht nur dort, ist es wichtig, eine Palette von Materialien bereitstellen zu können, die zwar dem Grunde nach für dieselben Zwecke verwendet werden können und dieselben physikalischen und mechanischen Eigenschaften besitzen, wobei diese Eigenschaften jedoch an spezifische, häufig sogar individuelle Anforderungen detailgenau angepasst werden müssen. Beispiele sind die Farbe bzw. Transluzenz von Überkronungen, die Bruchfestigkeit, der E-Modul, die Biegbarkeit beim DreiPunkt-Biegeversuch, die Matrixhydrophilie/-hydrophobie, die Reaktivität gegenüber Substraten oder weiteren Matrix- oder Komposit-Bestandteilen wie Zahngewebe, Co-Reaktanten oder Reaktionspartnern in lonomer-Kompositen. Hier haben minimale Veränderungen häufig eine große Wirkung. Kann der mit diesen Materialien arbeitende Spezialist, beispielsweise ein Zahnarzt oder ein Zahntechniker, auf eine abgestufte Palette der für seine Zwecke erforderlichen Materialien zurückgreifen, wird er in die Lage versetzt, für jede einzelne Applikation das genau dazu passende Material auszuwählen.

Die Herstellung eines hochästhetischen Zahnersatzes wurde bereits in DE 10 2011 054440 vorgeschlagen. Die dort offenbarten Materialien sind jedoch teilweise kommerziell nicht verfügbar oder aber relativ teuer in der Herstellung. Es besteht daher ein Bedarf, weitere Materialien bereitzustellen, die ohne die Verwendung der dort eingesetzten Substituenten und Füllstoffe auskommen und sich relativ einfach und kostengünstig in hoher Variabilität herstellen lassen.

In der DE 10 2011 054444 A1 wird vorgeschlagen, zur Herstellung von ästhetisch annehmbarem Zahnersatz eine Brechzahlanpassung der Füllstoffe an die vorgegebenen Matrixsysteme vorzunehmen. Dieser Vorschlag kann an Grenzen stoßen, wenn entsprechende Füllstoffe kommerziell nicht verfügbar sind oder nur unter hohem Aufwand hergestellt werden können. Gerade Füllstoffe mit kleiner Brechzahl sind sehr teuer.

In der zum Zeitpunkt der vorliegenden Anmeldung noch unveröffentlichten Patentanmeldung DE 10 2013 108 594.6 werden arylsubstituierte Silane und Kieselsäure(hetero)polykondensate sowie daraus oder damit hergestellte Massen beschrieben. Die Einführung von Arylgruppen erhöht die Brechzahl des aus den Ausgangsmaterialien hergestellten, vernetzten Endmaterials. Die genannten Silane und Kieselsäure(hetero)polykondensate enthalten, soweit sie nicht weiteren Umsetzungen unterzogen werden, eine Gruppe R², ausgewählt unter einer Hydroxygruppe oder einem freien Carbonsäurerest oder einem davon abgeleiteten Salz, sowie eine Arylgruppe, wobei die Arylgruppe über die Reaktion einer Thioverbindung, einer Aminoverbindung oder einem Phosphonsäurederivat mit einer C=C-doppelbindunghaltigen Verbindung über eine Thiol-en-Addition bzw. verwandte Addition in das Molekül eingeführt wird, d.h. unter Vernichtung einer Gruppe, die ansonsten für weitere Umsetzungen zur Verfügung stehen würde. Da die an die Doppelbindung addierende Verbindung im Unterschuss eingesetzt werden kann, erhält man bereits in diesem ersten Schritt bei Bedarf eine abgestufte Produktpalette.

Die Erfinder haben es sich nun zur Aufgabe gemacht, die Möglichkeiten der Abstufung verschiedener physikalischer und mechanischer Parameter ausgehend von nur einem einzigen Ausgangssystem noch feiner zu gestalten, wobei diese Parameter völlig unabhängig voneinander eingestellt werden können, selbst wenn die erhältliche Produktpalette durch die Umsetzung eines einzigen Ausgangssystems mit nur einem oder zwei Reaktionspartnern und/oder in nur einer oder ggf. zwei (dann zeitlich abgestuften) Umsetzungen erfolgt. Dabei sind als wesentliche Parameter die folgenden zu nennen: (1) hohe bzw. angepasste Brechzahl, um beispielsweise die Transluzenz von Dentalmaterialien, die aus den erfindungsgemäßen Systemen zusammen mit Füllstoffen erzeugt werden sollen, einstellen und die für Dentalmaterialien geforderte Ästhetik realisieren zu können, oder um eine hohe Transparenz von für optische Anwendungen benötigte Materialien zu erzielen; (2) mechanische Parameter wie E-Modul, Bruchfestigkeit oder Biegbarkeit (Flexibilität) und (3) eine geringe Härtungsschrumpfung. Hinsichtlich der zunehmenden Allergiediskussion, speziell beim Einsatz im Medizinsektor, ist ein Verzicht auf (meth)acrylat-basierte Monomere als Rückstand im Material natürlich ebenfalls wünschenswert.

In Lösung der genannten Aufgabe werden erfindungsgemäß organisch modifizierte Silane, Kieselsäure(hetero)polykondensate sowie daraus hergestellte Komposite (mit Füllstoffen gefüllte solche Polykondensate) sowie Verfahren zu deren Herstellung bereitgestellt, die zu einem gewissen, steuerbaren Anteil eine aromatische Gruppe aufweisen, welche über Sauerstoff, eine Carboxylgruppe, Stickstoff, Schwefel oder Phosphor an einen über Kohlenstoff an Silicium gebundenen Kohlenwasserstoffrest gebunden ist. Die Reaktion für die Einführung dieser Gruppe ist so gestaltet, dass gleichzeitig eine freie Hydroxygruppe entsteht. Durch die Einführung der aromatischen Gruppe wird also nicht, wie in DE 10 2013 108 594.6, eine reaktive Gruppe verbraucht und damit "vernichtet", sondern es wird eine neue reaktive Gruppe erzeugt. Dies gelingt dadurch, dass zur Einführung der aromatischen Gruppe eine cyclische Ethergruppe am Silan/Kieselsäure(hetero)polykondensat genutzt wird. Wird dieses Prinzip mit dem in DE 10 2013 108 594.6 angewandten Prinzip kombiniert, die Reaktionspartner der Silane/Kieselsäure(hetero)polykondensate im Unterschuss anzuwenden, enthalten die Produkte weiterhin einen beliebig einstellbaren Anteil an unverbrauchten cyclischen Ethergruppen. Diese sind ihrerseits hochreaktiv und können für die Einführung weiterer gewünschter Substituenten/ Gruppen genutzt werden, wobei sie ggf. mit der Hydroxygruppe in Konkurrenz treten können.

Durch geeignete Wahl der aromatischen Substituenten sowie der Menge, mit der sie in die Kieselsäure(hetero)polykondensate eingebaut sind, lässt sich die Brechzahl der ausgehärteten Harze bzw. der aus den Harzen durch organische Polymerisation erzeugten anorganisch-organischen Materialien genau steuern und damit eine Anpassung der Brechzahl des Materials an die Brechzahl von Füllstoffen erreichen, mit denen es ggf. gefüllt werden soll. Mit der Einstellung einer bestimmten Brechzahldifferenz lässt sich eine spezifische Transluzenz in gewünschter Weise erreichen, während eine identische oder fast identische Brechzahl des Matrixmaterials und des darin eingebetteten Füllstoffs in der Regel zu einer besonders hohen Transluzenz führt. Die freien Hydroxygruppen sowie auch möglicherweise vorhandene reaktive cyclische Ethergruppen ermöglichen die Anbindung weiterer funktionell wirksamer Gruppen an die Silane/Kieselsäure(hetero)polykondensate, beispielsweise von organisch polymerisierbaren Gruppen, durch die sich zusätzlich zum anorganischen Netzwerk des (Hetero)kondensats ein organisches Netzwerk aufbauen lässt und die gegebenenfalls ihrerseits wiederum unterschiedlichen Härtungsmechanismen unterliegen, so dass sich die mechanischen Eigenschaften des Produkts (z.B. der E-Modul, die Festigkeit und/oder die Härtungsschrumpfung) ebenfalls und unabhängig von der Brechzahl einstellen und steuern lassen. Wenn beide Reste nebeneinander vorhanden sind, können sie nebeneinander oder nacheinander gesteuert weiter umgesetzt werden, wodurch sich eine genaue Einstellung der jeweils gewünschten Parameter im Endprodukt erzielen lässt. Nicht weiter umgesetzte freie Hydroxygruppen sorgen für eine erhöhte Matrixhydrophilie; nicht weiter umgesetzte reaktive cyclische Ethergruppen können zu einem späteren Zeitpunkt polymerisiert werden oder lassen sich hydrolysieren, wobei zwei weitere Hydroxygruppen pro Ethergruppe entstehen, mit denen sich wiederum entweder die Matrixhydrophilie einstellen lässt oder die ihrerseits für die Ankopplung weiterer Gruppen zur Verfügung stehen.

Das Dokument WO 2013/053745 offenbart Silane, die dem vorliegenden Silan ähneln, jedoch anstelle eine Ar-Gruppe eine ethylenisch ungesättigte Gruppe aufweisen.

Die organisch modifizierten Kieselsäure(hetero)polykondensate der Erfindung können aus bereits entsprechend modifizierten Silanen durch gesteuerte hydrolytische Kondensation erzeugt werden, oder der oder die Schritte der Modifikation erfolgt an den bereits durch hydrolytische Kondensation erzeugten Kieselsäure(hetero)polykondensaten. Je nach eingeschlagenem Herstellungsweg können auch über diese unterschiedlichen Routen nochmals zusätzliche Abstufungen und Varianten erzeugt werden, was aus der nachstehenden, ausführlichen Beschreibung ersichtlich wird.

Wird demnach ein Silan mit einem Rest, der eine oder mehrere reaktive cyclische Ethergruppen enthält, oder wird ein Kieselsäure(hetero)polykondensat mit einer definierten molaren Menge an reaktiven cyclischen Ethergruppen als Ausgangssystem eingesetzt, erhält man bei der Umsetzung mit einer Verbindung, die mit der bzw. den cyclischen Ethergruppen reagiert und mit der die aromatische Gruppe eingeführt wird, ein Produkt, das die gleiche Anzahl an aromatischen Gruppen und an Hydroxygruppen enthält. Wird die Reaktion stöchiometrisch so geführt, dass nicht alle cyclischen Ethergruppen umgesetzt werden, erhält man ein Produkt, das zusätzlich solche Ethergruppen enthält. Diese Variabilität lässt sich in nur einem Reaktionsschritt und mit nur einem Reaktionspartner erreichen.

Nachstehend soll die Erfindung im Einzelnen näher erläutert werden.

Kernpunkte der Erfindung sind die Bereitstellung von Silanen der Formel (I)

R¹ₘR²ₙSiX₄₋ₘ₋ₙ, (I)

worin
R¹ ein über Kohlenstoff am Siliciumatom gebundener kohlenwasserstoffhaltiger, verzweigter oder unverzweigter Rest ist, der an mindestens einem seiner Kohlenstoffatome mit einer Gruppe der Formel

   (Ar)_{b}(W)ₐ-A-Y(R³)- (II)

   sowie optional mit einem oder mehreren weiteren Substituenten substituiert ist,
   worin Ar einen aromatischen Rest bedeutet, der mindestens eine Aryl- und/oder Heteroarylgruppe trägt oder daraus besteht,
   W ein substituierter oder unsubstituierter Kohlenwasserstoffrest ist, dessen Kette unterbrochen sein kann durch -S-, -O-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
   A entweder zweibindig ist und dann die Bedeutung -O-, -C(O)O-, -S-, -NR⁴- oder -P(O)ₑ(R⁴)_{c}(Z)_{d}- mit Z = OR⁴, c = 0 oder 1, d = 0 oder 1, (c+d) = 1 und e = 0 oder 1 hat oder dreibindig ist und -N= oder -P(O)ₑ= bedeutet, wobei die mit = bezeichneten Bindungen zwei Einzelbindungen darstellen,
   R⁴ ein beliebiger kohlenwasserstoffhaltiger Rest, vorzugsweise ein Alkylrest ist und im Rest -NR⁴- darüber hinaus außerdem Wasserstoff bedeuten kann,
   Y eine aus der Ringöffnung einer reaktiven cyclischen Ethergruppe hervorgegangene Alkylengruppe, z.B. der Zusammensetzung C₂H₃ oder C₃H₅, darstellt, die neben dem in der Formel (II) angegebenen Rest R³ weitere Substituenten tragen kann, aber nicht muss,
   R³ entweder OH ist oder -D-{B} oder -D-(W)ₐ(Ar)_{b} bedeutet, wobei D ausgewählt ist unter einer über Kohlenstoff an {B} oder an W oder, im Falle von a = 0, an Ar gebundenen Carboxylgruppe (Estergruppe), Ethergruppe, Urethangruppe und mit Hydroxy substituierten Ethylenoxygruppe und {B} ein organisch polymerisierbarer Rest ist, der mindestens eine organisch polymerisierbare C=C-Doppelbindung trägt,
   wobei Ar jeweils an W gebunden sein muss, sofern a ≠ 0 ist,
   a 0 oder 1 ist und dann, wenn A dreibindig ist, auch 2 sein kann, und
   b 1 oder, im Falle a = 1 und/oder für den Fall, dass A dreibindig ist und a die Anzahl der an A gebundenen Gruppen W angibt, eine ganze Zahl größer 1 sein kann,
R² ein über Kohlenstoff an das Silicium gebundener kohlenwasserstoffhaltiger Rest ist,
X eine unter hydrolytischen Bedingungen von Silicium abhydrolysierbare Gruppe oder OH ist,
m 1, 2 oder 3 ist, und
n 0 oder 1 oder 2 ist, mit der Maßgabe, dass m + n 1, 2 oder 3 sind,
und die Bereitstellung von organisch modifizierten Kieselsäure(hetero)polykondensaten, enthaltend über Kohlenstoff am Siliciumatom gebundene kohlenwasserstoffhaltige, verzweigte oder unverzweigte Reste R¹, die an mindestens einem Kohlenstoffatom mit einer Gruppe der Formel

   (Ar)_{b}(W)ₐ-A-Y(R³)- (II)

   sowie optional mit weiteren Substituenten substituiert sind,
   worin Ar einen aromatischen Rest bedeutet, der mindestens eine Aryl- und/oder Heteroarylgruppe trägt oder daraus besteht,
   W ein substituierter oder unsubstituierter Kohlenwasserstoffrest ist, dessen Kette unterbrochen sein kann durch -S-, -O-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
   A entweder zweibindig ist und dann die Bedeutung -O-, -C(O)O-, -S-, -NR⁴- oder -P(O)ₑ(R⁴)_{c}(Z)_{d}- mit Z = OR⁴, c = 0 oder 1, d = 0 oder 1, (c+d) = 1 und e = 0 oder 1 hat oder dreibindig ist und -N= oder -P(O)ₑ= bedeutet,
   R⁴ ein beliebiger kohlenwasserstoffhaltiger Rest, vorzugsweise ein Alkylrest ist und im Rest -NR⁴ darüber hinaus außerdem Wasserstoff bedeuten kann,
   Y eine aus der Ringöffnung einer reaktiven cyclischen Ethergruppe hervorgegangene Alkylengruppe, z.B. der Zusammensetzung C₂H₃ oder C₃H₅, darstellt, die neben dem in der Formel (I) angegebenen Rest R³ weitere Substituenten tragen kann, aber nicht muss,
   R³ entweder OH ist oder -D-{B} oder -D-(W)ₐ(Ar)_{b} bedeutet, wobei D ausgewählt ist unter einer über Kohlenstoff an {B} gebundenen Carboxylgruppe (Estergruppe), Ethergruppe, Urethangruppe und mit Hydroxy substituierten Ethylenoxygruppe, und {B} ein organisch polymerisierbarer Rest ist, der mindestens eine organisch polymerisierbare C=C-Doppelbindung trägt,
   wobei Ar jeweils an W gebunden sein muss, sofern a ≠ 0 ist,
   a 0 oder 1 ist und dann, wenn A dreibindig ist, auch 2 sein kann, und
   b 1 oder, im Falle a = 1 und/oder für den Fall, dass A dreibindig ist, eine ganze Zahl größer 1 sein kann.

Der Ausdruck "kohlenwasserstoffhaltiger Rest" umfasst Reste mit durchgehender und solche mit unterbrochener Kohlenwasserstoffkette, wobei die Unterbrechung z.B. durch mindestens eine Gruppe bewirkt sein kann, wie sie als unterbrechende Gruppe für den Rest W oben angegeben ist.

Die Kieselsäure(hetero)polykondensate können ungefüllt oder mit Füllstoff befüllt (als "Komposit") vorliegen.

Unter dem nachfolgend verwendeten Begriff "Systeme" sollen Silane, Kieselsäure(hetero)-polykondensate und Komposite zu verstehen sein. Der Ausdruck "erfindungsgemäße Systeme" bezeichnet erfindungsgemäße Silane, Kieselsäure(hetero)polykondensate und/oder Komposite.

Bei den erfindungsgemäßen organisch modifizierten Kieselsäure(hetero)polykondensaten kann es sich um ausschließlich aus Silanen aufgebauten Kieselsäurepolykondensaten oder um solche handeln, die neben Silicium andere Metallatome M cokondensierter Verbindungen aufweisen, beispielsweise M = B, Al, Ti, Zn und/oder weitere Übergangsmetallatome, wie dem Grunde nach aus dem Stand der Technik bekannt. Solche Kondensate werden üblicherweise - und auch vorliegend - als Kieselsäure(hetero)polykondensate bezeichnet, wobei der Einschub "hetero" für die Anwesenheit co-kondensierter Metallatome steht. Des Weiteren kann es sich um vollständig oder um nur teilweise vernetzte Kondensate von Silanen und ggf. Metallverbindungen, insbesondere Akoxid-Metallverbindungen, handeln. Wenn die Kondensate nur teilweise vernetzt sind, weisen sie noch an Silicium und/oder Metall gebundene, hodrolytisch kondensierbare Reste oder freie Hydroxygruppen auf, die eine (spätere) weitergehende anorganische Vernetzung unter Ausbildung zusätzlicher Si-O-Si- oder Si-O-M- oder M-O-M-Brücken zulassen. zulassen. Wird der Ausdruck "Kieselsäurepolykondensat" verwendet, so umfasst dieser Ausdruck erfindungsgemäß sowohl Hetero- als auch Teil-Kondensate, sofern dies nicht aufgrund des Kontextes ausgeschlossen ist.

Bei den verzweigten oder unverzweigten Resten R¹ in den oben genannten Silanen und Kieselsäure(hetero)polykondensaten handelt es sich um Kohlenwasserstoffreste, beispielsweise Alkylreste, die beliebig durch Heteroatome oder Kupplungsgruppen oder andere, Heteroatome enthaltende Gruppen unterbrochen sein können. Beispiele sind Unterbrechungen durch -S-, -O-, -NH-, -C(O)O-, -NHCH(O)-, -C(O)NH- -NHC(O)O-, -C(O)NHC(O)-, -NHC(O)NH-, -S(O)-, -C(S)O-, -C(S)NH-, -NHC(S)-, -NHC(S)O- und dergleichen. Da es für die Zwecke der Erfindung auf die Struktur dieser Reste nicht ankommt, soweit nicht deren beschriebene Substituenten betroffen sind, kann der Fachmann diesbezüglich eine beliebige Auswahl treffen.

Die Position des Substituenten mit der Formel (II) am Kohlenwasserstoffrest R¹ ist frei wählbar.

Der bzw. die Reste R¹ im Silan bzw. im Kieselsäure(hetero)polykondensat sind vorzugsweise mit einer Gruppe der Formel (II), gelegentlich aber auch mit zwei oder noch mehr solchen Gruppen substituiert. In der Formel (II) bedeutet Ar einen aromatischen Rest, der mindestens eine Aryl- und/oder Heteroarylgruppe trägt oder daraus besteht. Die Aryl- und Heteroarylgruppen des aromatischen Restes von Ar können unsubstituiert sein; sie können statt dessen einfach oder mehrfach substituiert sein, wobei die Substituenten vorzugsweise ausgewählt sind unter Alkyl, Alkoxy, Alkylmercapto, Alkoxyalkylen, Alkylthioalkylen, Alkylcarbonyl, Alkylketoalkylen (auch als Alkylcarbonylalkylen zu bezeichnen), Alkylcarboxyl, Alkylcarboxylalkylen, Nitril und Halogen (insbesondere Chlor, Brom, lod). Eine Substitution mit Säuregruppen (z.B. Boronsäure-, Carbonsäure-, Phosphin- oder Phosphonsäure- oder Sulfonsäuregruppen) oder Hydroxygruppen ist dagegen wenig wünschenswert und sollte nach Möglichkeit vermieden werden. Enthält der aromatische Rest mehrere Arylgruppen, so können diese durch andere Gruppen, vorzugsweise ausgewählt unter -R^{x}-, -(R^{x})ₓ-C(O)-(R^{y})_{y}-, -(R^{x})ₓ-S-(R^{y})_{y}-, -(R^{x})ₓ-S(O)-(R^{y})_{y}-, -(R^{x})ₓ-S(O)₂-(R^{y})_{y}-, -(R^{x})ₓ-O-(R^{y})_{y}- und -(R^{x})ₓ-C(O)₂-(R^{y})_{y}-, unterbrochen sein, worin R^{x} und R^{y} gleich oder verschieden sind und eine vorzugsweise kurzkettige Alkylengruppe (z.B. mit C₁-C₆, darunter -CH₂- und -C(CH₃)₂-) bedeuten und die Indices x und y unabhängig voneinander 0 oder 1 bedeuten Dabei hat in vielen Fällen sowohl x als auch y die Bedeutung 0. Bisphenol-A-, Benzophenon-, Diphenylsulfon-, Diphenylsulfid- oder Diphenylether-Strukturen sind Beispiele hierfür. Alternativ können die vorhandenen Arylgruppen über eine Bindung miteinander verknüpft (wie z.B. im Biphenyl-, Terphenyl- oder Bipyridyl-Rest) oder kondensiert (wie z.B. im Naphthyl-, Anthracen-, Chrysen-Rest) vorliegen. Die voranstehenden Beispiele betrafen Aromaten ohne Heteroatome im Ring. Als Heteroarylgruppen seien beispielhaft die folgenden genannt: die Thiophen-, Furan-, Pyridin- und die Chinolin-Struktur.

Wenn der Rest A zweibindig ist, hat der Index a in der Gruppe mit der Formel (II) die Bedeutung 0 oder 1, wenn er dreibindig ist, kann a auch 2 sein. Das bedeutet, dass die Gruppe W einfach oder, im Falle eines dreibindigen Restes A, zweifach vorhanden sein oder aber auch fehlen kann. Im Falle des Fehlens der Gruppe W sind der oder die Reste Ar direkt an A angebunden. Dabei können je nachdem, ob A zweibindig oder dreibindig ist, ein oder zwei Reste Ar an A angebunden sein. Für a = 0 muss b daher 1 oder 2 sein. Bei Anwesenheit von W kann b ebenfalls 1 oder 2 sein, kann aber auch größer sein, beispielsweise 3, 4, 5 oder 6 oder sogar eine hoch höhere Zahl bedeuten. Dabei muss mindestens ein Rest Ar an jeder Gruppe W gebunden sein; es können jedoch auch mehrere (zwei oder noch mehr) Reste pro Gruppe W sein.

Y stellt, wie oben angegeben, eine aus der Ringöffnung einer reaktiven cyclischen Ethergruppe hervorgegangen Alkylengruppe dar. Da es bei einem asymmetrischen cyclischen Ether notwendigerweise zwei Möglichkeiten der Ringöffnung gibt, kann diese Alkylengruppe in isomerer Form auftreten. Beispielsweise kann in dem einfachen Fall, wenn es sich bei der cyclischen Ethergruppe um ein nicht zusätzlich substituiertes Epoxid (Oxiran) handelt, die entstehende C₂H₃R³-Gruppe die Form eines mit R³ substituierten Ethylens (mit beiden Kohlenstoffatomen in der Hauptkette) oder eines mit R³-CH₂ substituierten Methylens (mit nur einem der beiden Kohlenstoffatomen in der Hauptkette) haben, wie auch weiter unten anhand eines Beispiels erläutert wird. Wenn die cyclische Ethergruppe an einen Kohlenwasserstoffring gebunden war, bleibt dieser Ring erhalten. So kann Y eine mit R³ substituierte Cycloalkylengruppe sein, z.B. eine Cyclohexylengruppe.

R⁴ ist in allen vorstehenden Zusammenhängen ein substituierter oder unsubstituierter kohlenwasserstoffhaltiger Rest, häufig ein Kohlenswasserstoffrest, beispielsweise ein Aryl-, Alkylaryl oder Arylalkylrest und vorzugsweise ein substituierter oder - vorzugsweise - unsubstituierter Alkylrest mit stärker bevorzugt 1 bis 6 Kohlenstoffatomen. Außer in der Formel -P(O)ₑ(R⁴)_{c}(Z)_{d}- kann R⁴ darüber hinaus in allen diesen Zusammenhängen auch Wasserstoff bedeuten (die Ausnahme gilt auch für die Definition Z' = OR⁴; auch hier darf R⁴ nicht Wasserstoff bedeuten).

Der Rest {B} trägt, wie erwähnt, mindestens eine organisch polymerisierbare, d.h. in der Regel nicht-aromatische C=C-Doppelbindung. Diese Doppelbindung(en) kann/können beispielsweise als ein Bestandteil von Styryl-,Vinyl- oder Allylgruppen oder von einer Michael-Addition zugänglichen Gruppen wie (Meth)Acryl- oder Norbornenylgruppen vorliegen. Häufig besitzt der organisch polymerisierbare Rest zwei, manchmal auch drei, vier oder noch mehr solcher C=C-Doppelbindungen. In spezifischen Ausführungsformen ist er oder weist er mindestens eine (Meth-)Acrylgruppe auf. Die (Meth-)Acrylsäure-Derivate umfassen die Säuren selbst, ggf. in aktivierter Form, Ester, Amide, Thioester und dgl.. (Meth-)Acrylatgruppen sind darunter bevorzugt. Der Rest {B} enthält mindestens zwei und vorzugsweise bis zu ca. 50, ggf. aber auch noch mehr Kohlenstoffatome; er weist ein geradkettiges, verzweigtes oder cyclisches Kohlenwasserstoff-Skelett auf, das durchgängig oder durch Kupplungsgruppen, z.B. durch -S-, -O-, -NH-, -C(O)O-, -NHCH(O)-, -C(O)NH-, -NHC(O)O-, -C(O)NHC(O)-, -NHC(O)NH-, -S(O)-, -C(S)O-, -C(S)NH-, -NHC(S)- oder -NHC(S)O-, unterbrochen sein kann.

Der Ausdruck "(Meth-)Acryl..." , soll vorliegend in allen Ausgestaltungen der Erfindung die Bedeutung haben, dass es sich jeweils um die entsprechende Acryl- oder die entsprechende Methacryl-Verbindung oder ein Gemisch von beiden handeln kann.

Unter "hydrolytisch kondensierbaren" Resten sind alle aus dem Stand der Technik bekannten Reste zu verstehen, die unter hydrolytischen Bedungungen zur Ausbildung von Si-O-Si-, Si-O-M- bzw. M-O-M-Brücken führen, z.B. Halogenide oder Alkoxide, wobei Alkoxide bevorzugt sind.

In den Silanen der Erfindung ist das Verhältnis der Summe der über Kohlenstoffatome an Silicium gebundenen Reste (R¹ + R²) (d.h. die Summe aus m und n) zu den hydrolytisch kondensierbaren Resten X bzw. zu OH im Grunde nicht kritisch; der Fachmann weiß, dass im Falle von drei hydrolytisch kondensierbaren Resten bzw. OH-Gruppen ein sehr dichtes anorganisches Netzwerk entsteht, während im Falle von zwei solchen Resten bzw. OH-Gruppen vorwiegend Ketten und/oder Ringe gebildet werden. Beim ausschließlichen Vorhandensein von nur einem solchen Rest / einer OH-Gruppe können nur Dimere gebildet werden. Es werden daher je nach Anwendungszweck geeignete Varianten bzw. Kombination (z.B. aus Silanen mit zwei Resten X und Silanen mit drei Resten X) hierunter ausgewählt; in der Regel sind Ausgangssilane mit 2 hydrolytisch kondensierbaren Resten bzw. OH-Gruppen gut für die Erfindung geeignet.

In einer ersten Ausgestaltung handelt es sich bei den erfindungsgemäßen Silanen der Formel (I) um solche, in denen R³ OH bedeutet. Diese sind erhältlich durch Umsetzen eines Silans mit der Formel

R^{1'}ₘR²ₙSiX₄₋ₘ₋ₙ, (III),

worin R², X, n und m wie für Formel (I) definiert sind und R¹' ein über Kohlenstoff am Siliciumatom gebundener kohlenwasserstoffhaltiger, verzweigter oder unverzweigter Rest ist, der an mindestens einem seiner Kohlenstoffatome eine reaktive, cyclische Ethergruppe sowie ggf. einen oder mehrere weitere Substituenten trägt, mit einer Verbindung der Formel (1)

(Ar)_{b}(W)ₐ-Q (1),

worin Ar, W, a und b wie oben für den Substituenten der Formel (II) definiert sind und Q ausgewählt ist unter-OH, -C(O)OH, -SH, -NHR⁴, -PH(O)ₑ(R⁴)_{c}(Z)_{d} mit Z = OR⁴, c = 0 oder 1, d = 0 oder 1 und (c+d) = 1, HN= oder HP(O)=, wobei Z und R⁴ wie oben für Formel (II) definiert sind, wobei in diesem Fall R⁴ bevorzugt Alkyl ist oder die Bedeutung Ar wie oben definiert hat und z.B. ein Arylrest ist. Ein spezielles Beispiel für eine Verbindung (1) mit a = 0 und b = 2 ist Diphenylphosphinoxid, wobei in diesem Beispiel Q HP(O)= bedeutet. Die Umsetzung mit der Verbindung der Formel (1) wird nachstehend als Reaktion 1 bezeichnet.

Unter dem Ausdruck "reaktive, cyclische Ethergruppe" soll erfindungsgemäß bevorzugt eine Glycidylgruppe (Epoxidgruppe) zu verstehen sein; er kann aber auch eine cyclische Ethergruppe mit vier Ringgliedern, also ein Oxetan bezeichnen. Die Glycidyl- bzw. Oxetangruppe kann unsubstituiert, mit einer, zwei oder, im Falle einer Oxetangruppe, drei Alkylgruppen, mit einer vicinal bindenden Alkylengruppe (z.B. einer Hexylengruppe) oderwiederum nur im Falle einer Oxetangruppe - mit einer vicinal bindenden Alkylengruppe und einer Alkylgruppe substituiert sein. Silane mit über Kohlenstoff gebundenen Kohlenwasserstoffresten, die solche Gruppen tragen, sind in großer Zahl bekannt und teilweise auch käuflich, beispielsweise 3-Glycidyloxypropyltrimethoxysilan, 3-Glycidyloxypropylmethyldimethoxysilan, 3-Glycidyloxypropyldimethylethoxysilan, 3-Glycidyloxypropylmethyldiethoxysilan, 3-Glycidyloxypropyltriethoxysilan, 3-Glycidyloxypropyldimethylmethoxysilan, 3-Glycidoxypropylmethyl-diisoprenoxysilan, [2-(3,4-Epoxy-4-methylcyclohexyl)propyl]-methyldiethoxysilan oder Verbindungen der Formel IX aus DE 4416857 C1, darunter 2-(3,4-Epoxycyclohexyl)-ethyltriethoxysilan und 2-(3,4-Epoxycyclohexyl)ethyltrimethoxysilan mit den Formeln:

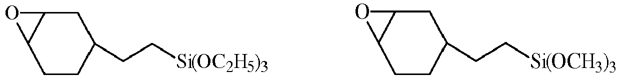

Die Additionsreaktion einer Verbindung (1) an ein Silan (III) und die dabei möglicherweise entstehenden Isomere seien anhand der Umsetzung der Carbonsäure Benzoesäure an Glycidyloxypropyltmethyldiethoxysilan erläutert:

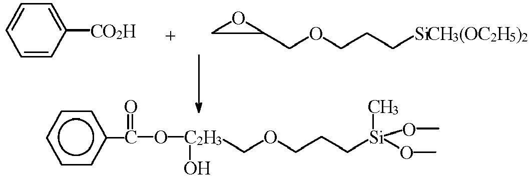

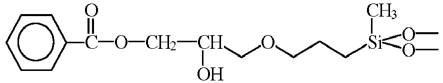

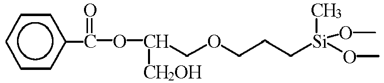

Das Produkt besteht aus den beiden untenstehenden Isomeren, wobei das erste begünstigt ist.

Die Gruppe "C₂H₃OH" soll diese beiden Varianten umfassen. Wird anstelle der Glycidyloxypropylgruppe die oben gezeigte 3,4-Epoxycyclohexylgruppe eingesetzt, entsteht ein Produkt, bei dem die beiden Kohlenstoffatome der ehemaligen Epoxygruppe Bestandteil einer Cycloalkenylgruppe (hier der Cyclohexylgruppe) sind. Die cyclische Gruppe kann dabei in verschiedenen Isomeren entweder über zwei oder nur über ein Kohlenstoffatom an die Hauptkette angebunden sein.

Durch die Reaktion von Q = COOH mit der reaktiven cyclischen Ethergruppe von R¹', hier der Glycidylgruppe, entsteht die Gruppierung -A-Y(R³)- mit A = C(O)O, Y = C₂H₃ und R³ = OH.

Beispiele für Verbindungen mit der Formel (1) mit Q = COA' und A' = OH sind Benzoesäure, Biphenyl-2-bzw. 4-carbonsäure, 9-Anthracencarbonsäure, Naphthalin-1 bzw- 2-carbonsäure, 4-(1-Pyrenyl)butansäure, 1-Pyrenylcarbonsäure, Phenoxy-essigsäure, 2- bzw. 3- bzw. p-Phenoxy-benzoesäure, p-Terphenyl-4-carbonsäure, 2- bzw. 3-Phenoxy-propionsäure, 2 bzw. 4-(Methylphenoxy)essigsäure, 4-(4-Methylphenoxy)-benzoesäure und 2-Naphthylessigsäure sowie, als Beispiele für Heteroaromaten, 3-(2-Furyl)-propionsäure, Furan-3-carbonsäure, 2-Picolinsäure, 3- bzw. 6-Methylpicolinsäure, 2- bzw. 3-Thiophenessigsäure und 2- bzw. 3-Thiophencarbonsäure.

Wenn Q = OH ist, ensteht eine Struktur mit A = O.

Beispiele für Verbindungen der Formel (1) mit Q = OH sind Phenol, 1 bzw. 2-Phenylethanol, 3-Phenoxy-benzylakohol, 3 bzw. 4-Phenoxy-phenol, 1-Phenoxy-2-propanol, Biphenyl-2 bzw.4-methanol, 9-Anthracenmethanol, Anthracen-1- bzw. 2-ol, 2-(1 bzw. 2-Naphthyl)-ethanol, 1,3-Diphenoxy-2-propanol und {[2-Hydroxy-3-(phenylsulfanyl)propyl]sulfanyl}benzen sowie, als Beispiele für Heteroaromaten, N-(2-Hydroxyethyl)carbazol, 2-Hydroxycarbazol, 3-(Hydroxymethyl)furan, 2-(Hydroxymethyl)furan, 2- bzw. 3-Thiophenmethanol, 2- bzw. 3-Thiophenethanol, 4-(3-Thienyl)-phenol.

Beispiele für Verbindungen mit der Formel (1) mit anderen Resten Q sind Thiophenol, 1-Naphthalenthiol, 2-Naphthalenthiol, 4-Phenylthiophenol, 4-Terphenylthiol, 2-, 3- und 4-Thiokresol, N-Methyl-4-biphenylamin, N-Methyl-1-naphthylamin, N-Methyl-2-naphthylamin, Carbazol, 4-(3-Thienyl)-anilin, 2-Aminoanthracen, 2-Thiophenmethylamin, Anilin, 4-Phenylsulfanylanilin, 4-Benzylanilin, 1-Aminonaphthalin, 2-Aminonaphthalin, 6-Aminochrysen, 2-, 3- und 4-Aminobenzophenon, 3,- 5- und 6-Aminochinolin, Pyrrol, 3- bzw. 4-Aminoacetophenon, Phosphonsäurederivate wie Diphenylphosphit H-P(O)(OPh)₂ und Phosphinoxide wie Diphenylphosphinoxid H-P(O)(Ph)₂. Mit Q = SH ensteht eine Struktur mit A = S, mit Q = NHR⁴ entsteht eine Struktur mit A = N(R⁴), und mit Q = PH(O)ₑ(R⁴)_{c}(Z)_{d} entsteht eine Struktur mit A = P(O)ₑ(R⁴)_{c}(Z)_{d}.

Zur Herstellung der Kieselsäure(hetero)polykondensate mit Resten R¹ wie oben definiert und R³ = OH können entweder Silane der Formel (I), ggf. in Gegenwart weiterer Silane und/oder hydrolytisch kondensierbarer Metallverbindungen, einer hydrolytischen Kondensation unterworfen werden, oder es wird ein Kieselsäure(hetero)polykondensat, das Reste R¹' wie oben für Formel (III) definiert aufweist, mit einer Verbindung der Formel (1) wie oben definiert umgesetzt.

Dabei ist es nicht notwendig, dass jedes Siliciumatom des Kieselsäure(hetero)polykondensats einen oder mehrere Reste R¹' aufweist, vielmehr genügt es, wenn ein Teil der Siliciumatome Reste R¹' trägt. Solche Polykondensate sind auf verschiedene Weise erhältlich. So ist es möglich, das Polykondensat nicht ausschließlich aus Silanen zu erzeugen, die mindestens eine reaktive cyclische Ethergruppe aufweisen, sondern in Gegenwart weiterer Silane ohne solche Gruppen, die bei der Ausbildung des Kieselsäure(hetero)polykondensats co-kondensiert werden. Auch kann die cyclische Ethergruppe im Unterschuss, bezogen auf die Siliciumatome, in das Kieselsäure(hetero)polykondensat eingeführt werden.

Damit ergibt sich bereits für das zu wählende Ausgangsmaterial die Möglichkeit, das molare Verhältnis von reaktiver cyclischer Ethergruppe zu Siliciumatomen in weiten Bereichen einzustellen. Günstig sind mindestens 0,2 Mol cyclische Ethergruppe pro Mol Silicium. Eine brauchbare Obergrenze ist schwer anzugeben, da sie vom gewünschten Einsatzzweck abhängt; es ist aber durchaus möglich, Systeme einzusetzen, die bis zu drei, gegebenfalls sogar noch mehr als drei Mol cyclische Ethergruppen pro Mol Silicium enthalten. Wird dieses Ausgangsmaterial mit einer Menge an Verbindung (1) umgesetzt, mit der alle vorhandenen cyclischen Ethergruppen zur Reaktion gebracht werden, entsteht ein erfindungsgemäßes System, das pro Mol Silicium die entsprechende molare Menge an OH-Gruppen sowie an Gruppen (Ar)_{b}(W)ₐ enthält, also im Rahmen der obengenannten Mengenverhältnisse 0,2 bis 3 oder sogar mehr als 3 Mol OH pro Mol Silicium sowie 0,2 bis 3 oder sogar mehr als 3 Mol Ar, multipliziert mit dem Faktor b. Dabei ist zu berücksichtigen, dass der Faktor b 1, 2 oder größer 2 sein kann; er kann jedoch auch stufenlos eingestellt werden, indem nämlich eine Mischung von Verbindungen (1) eingesetzt wird, in denen b unterschiedliche Werte besitzt. Daraus wird ersichtlich, dass die in die erfindungsgemäßen Systeme eingeführten Gruppen Ar in ihrer molaren Menge, relativ zur Molmenge von Si, in einer großen Variation vorliegen können, während die Menge an freien Hydroxygruppen zu Silicium die Variationsbreite besitzt, die die Menge an cyclischen Ethergruppen im Ausgangsmaterial hatte, und gegenüber der Menge an Ar über den Faktor b in der Verbindung (1) gesteuert werden kann, so dass sie relativ zu Ar im molaren Verhältnis von 1 : b vorliegt.

Das Silan der Formel (I) oder das entsprechende Kieselsäure(hetero)polykondensat muss jedoch nicht notwendigerweise mit einer solchen Menge an Verbindung (1) umgesetzt werden, dass alle reaktiven cyclischen Ethergruppen damit reagieren (Überschüsse an Verbindung (1) sollten keinesfalls angewendet werden, denn dann würde damit die bei der Ringöffnung entstehende, weniger reaktive OH-Gruppe angegriffen werden). Stattdessen kann mit einem Unterschuss an Verbindung (1) gearbeitet werden, bezogen auf die vorhandenen reaktiven cyclischen Ethergruppen. Wenn das molare Verhältnis von Verbindung (1) zu den vorhandenen reaktiven cyclischen Ethergruppen α < 1 ist, wird nur ein Teil dieser Gruppen umgesetzt. Die restlichen cyclischen Ethergruppen bleiben erhalten; sie können aber auch, z.B. im Rahmen einer hydrolytischen Kondensationsreaktion des Produkts, wenn die Reaktion mit Verbindung (1) an einem Silan durchgeführt wurde, unter Ausbildung von zwei vicinalen OH-Gruppen geöffnet werden (es ist anzumerken, dass die hydrolytische Kondensation jedoch auch so geführt werden kann, dass die Ringe geschlossen bleiben, wie der Fachmann weiß). Eine derartige Umsetzung eines Silans der Formel (III) mit einem Unterschuss (einer molaren Menge α) an Verbindung (1) sei nachstehend anhand der Reaktion eines Mols 3-Glycidyloxypropylmethyldiethoxysilan mit einem Unterschuss von α Mol Benzoesäure gezeigt:

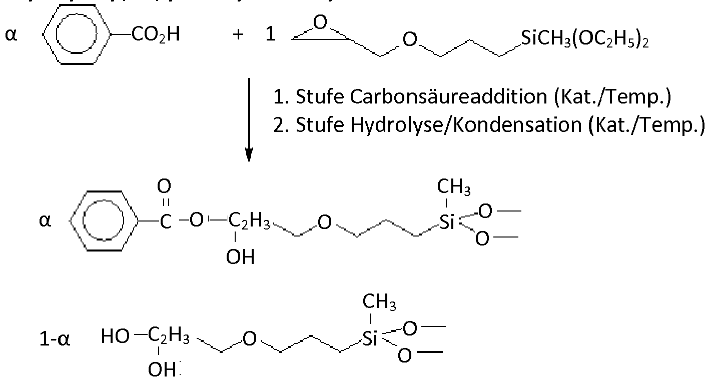

Es entsteht dann ein Silangemisch bzw. Kieselsäure(hetero)polykondensat, dessen Siliciumatome teilweise mit dem Substituenten R¹ (mit Ar = Phenyl, a = 0, A = C(O)O und Y = C₂H₃R³ mit R³ = OH) und teilweise mit einem Substituenten R¹" substituiert sind, der sich vom Substituenten R¹' dadurch unterscheidet, dass an die Stelle der reaktiven, cyclischen Ethergruppe, hier der Glycidylgruppe, eine C₂- oder C₃-Gruppe getreten ist, die zweifach mit einer Hydroxygruppe substituiert ist. Da pro Mol umgesetzter Verbindung (1) ein Mol Hydroxygruppen entsteht und pro Mol nicht umgesetztem Ausgangssilan zwei Mol Hydroxygruppen gebildet werden, lässt sich über das Verhältnis von Verbindung (1) zu den Glycidylgruppen die Anzahl an vorhandenen OH-Gruppen (und damit ggf. die Matrixhydrophilie, vor allem aber die Anzahl der für weitere Reaktionen bereitstehenden Gruppen) auf zwischen 1 und 2 Mol pro Mol der ursprünglichen Glycidylgruppen einstellen.

Werden für diese Reaktion Silane oder Kieselsäure(hetero)polykondensate eingesetzt, die am Rest R¹' mehr als eine reaktive cyclische Ethergruppe tragen, entstehen Systeme mit Resten R¹, die nicht nur mit mindestens einer Gruppe der Formel (II), d.h. mit (Ar)_{b}(W)ₐ-A-Y(R³)- (mit R³ = OH) substituiert sind, sondern auch mit einer C₂- oder C₃-Gruppe, die zwei vicinal zueinander stehende Hydroxygruppen aufweist.

Wenn mit einem Unterschuss an Verbindung (1) gearbeitet wird, ist es bevorzugt, mindestens 20 Mol-% davon, bezogen auf die Anzahl der reaktiven cyclischen Ethergruppen, einzusetzen, um eine genügend hohe Anzahl an Substituenten Ar in die Silane/Kieselsäure(hetero)polykondensate einzufügen. Stärker bevorzugt beträgt die molare Menge mindestens 30%, und noch stärker bevorzugt mindestens 50%.

Alternativ kann, wie oben angemerkt, so gearbeitet werden, dass der Ring der nicht umgesetzten cyclischen Ether-Gruppen erhalten bleibt. Dies gilt für alle oben diskutierten Umsetzungen; es bedeutet, dass der Rest R¹ in den erfindungsgemäßen Systemen neben der Gruppe der Formel (II) als Substituenten auch mit einer (ggf. sogar mehreren) reaktiven cyclischen Ethergruppen substituiert sein kann. Diese Gruppen stehen für eine spätere, meist kationische Polymerisation (unter Ausbildung von Polyether-Strukturen, zumeist Polyoxyethylen- oder -propylenketten) zur Verfügung, was weiter unten näher erläutert wird. Alternativ können sie zu weiteren Umsetzungen genutzt werden, beispielsweise mit (insbesondere sekundären) Diaminen unter Ausbildung von jeweils zwei Aminogruppen enthaltenden Verbrückungen zwischen zwei Strukturen, die jeweils aus einer cyclischen Ethergruppe hervorgegangen sind.

Wird mit einem Unterschuss α an Verbindung (1) gearbeitet, lässt sich eine weitere Abstufung erreichen, ohne dass ein zweiter Reaktionsschritt durchgeführt werden muss, indem als Reaktionspartner des Silans bzw. des Kieselsäure(hetero)polykondensats neben der Verbindung mit der Formel (1) eine Verbindung mit der Formel (2)

{B}-Q' (2)

eingesetzt wird, worin Q' ausgewählt ist unter -OH und -C(O)OH und {B} ein organisch polymerisierbarer Rest ist, der mindestens eine nichtaromatische C=C-Doppelbindung trägt, wobei diese Doppelbindung(en) beispielsweise als ein Bestandteil von Styryl-,Vinyl- oder Allylgruppen oder von einer Michael-Addition zugänglichen Gruppen wie (Meth)Acryl- oder Norbornenylgruppen vorliegen kann/können. Häufig besitzt der organisch polymerisierbare Rest zwei, manchmal auch drei, vier oder noch mehr C=C-Doppelbindungen; er kann offenkettig sein oder mindestens einen Kohlenstoffcyclus oder Heterocyclus aufweisen. In spezifischen Ausführungsformen ist er oder weist er mindestens eine (Meth-)Acrylgruppe auf. Die (Meth-) Acrylsäure-Derivate umfassen die Säuren selbst, ggf. in aktivierter Form, Ester, Amide, Thioester und dgl.. (Meth-)Acrylatgruppen sind darunter bevorzugt. Der Rest {B} enthält mindestens zwei und vorzugsweise bis zu ca. 50, ggf. aber auch noch mehr Kohlenstoffatome; er weist ein geradkettiges, verzweigtes oder cyclisches Kohlenwasserstoff-Skelett auf, das durchgängig oder durch Kupplungsgruppen, z.B. durch -S-, -O-, -NH-, -C(O)O-, -NHCH(O)-, -C(O)NH-, -NHC(O)O-, -C(O)NHC(O)-, -NHC(O)NH-, -S(O)-, -C(S)O-, -C(S)NH-, -NHC(S)- oder -NHC(S)O-, unterbrochen sein kann. Die Umsetzung mit der Verbindung (2) wird nachstehend unabhängig davon, ob sie als Konkurrenzreaktion zur Umsetzung mit der Verbindung (1) oder, wie nachstehend näher erläutert wird, separat durchgeführt wird, als Reaktion 2 bezeichnet.

Die Verbindung mit der Formel (2) besitzt ähnlich wie die Verbindung mit der Formel (1) eine Gruppe (Q' anstelle von Q in (1)), die mit cyclischen Ethergruppen reagieren kann, so dass sie mit letzterer um die Reaktion mit den cyclischen Ethergruppen konkurriert. Jedoch wird durch die Verbindung (2) keine aromatische Gruppe eingeführt, sondern - über eine weitere Gruppe Y-A"- - der Rest {B}, dessen nichtaromatische, organisch polymerisierbare C=C-Doppelbindung(en) einer organischen Polymerisationsreaktion (Polyaddition) zugänglich ist/sind und/oder die Anbindung weiterer funktioneller Gruppen ermöglichen. Wird auf Silan-Ebene gearbeitet, entsteht eine Mischung von Silanen mit Resten R¹', die eine Gruppe der Formel (II) mit R³ = OH tragen, mit Silanen der Formel (IV)

R^{1*}ₘR²ₙSiX₄₋ₘ₋ₙ, (IV)

worin R^{1*} ein über Kohlenstoff am Siliciumatom gebundener kohlenwasserstoffhaltiger, verzweigter oder unverzweigter Rest ist, der am mindestens einem seiner Kohlenstoffatome mit einer Gruppe {B}-A"-Y(OH)- substituiert ist, worin {B} und Y wie für die Formel (II) in Anspruch 1 definiert sind und A" ausgewählt ist unter -C(O)O- und -O-.

Die Verbindung mit der Formel (2) wird ebenfalls im Unterschuss, bezogen auf die reaktiven cyclischen Ethergruppen, eingesetzt. Dieser Unterschuss β kann so gewählt sein, dass die molare Menge (α + β) im Wesentlichen oder genau der molaren Menge an cyclischen Ether-gruppen entspricht. Dann entsteht ein Gemisch an Silanen bzw. ein Kieselsäure(hetero)polykondensat, bei dem man die Relation der Gruppen Ar zu den Resten {B} je nach Wunsch durch die Wahl des Verhältnisses der beiden Verbindungen zueinander genau steuern und einstellen kann. Die dabei hinzunehmende Verringerung der Menge an Gruppen Ar kann dabei ggf. durch entsprechende Wahl der Verbindung (1) (z.B. eine solche, in der b = 2 oder noch größer ist) oder durch eine hohe verfügbare Anzahl an cyclischen Ethergruppen kompensiert werden. Die Menge an Ar zu den über die Verbindung (2) eingeführten Doppelbindungen lässt sich über das Verhältnis von b in der Verbindung (1) zur Anzahl der organisch polymerisierbaren C=C-Doppelbindungen in {B} steuern.

Beispiele für die Verbindung (2) sind Säuren mit isolierten C=C-Doppelbindungen wie Allylessigsäure, 5-Norbornen-2-carbonsäure, Ölsäure bzw. Elaidinsäure (cis- bzw. trans-Oktadecen-9-säure), Oktadecen-9,12-säure und Oktadecen-9,12,15-säure, Säuren mit aktivierten C=C-Doppelbindungen wie Acrylsäure, Methacrylsäure, die Mono(meth)acryloyloxyethyl- oderpropylester der Maleinsäure, der Methylmaleinsäure, der Bernsteinsäure, der Glutarsäure, der Adipinsäure, der 1,2-Cyclohexandicarbonsäure und der 5-Norbornendicarbonsäure. Als Säuren mit mehreren C=C-Doppelbindungen können beispielsweise Reaktionsprodukte (Ester) des zweifach mit (Meth-)Acrylsäure veresterten Glycerins mit einer Disäure oder einem entsprechenden Anhydrid fungieren, beispielsweise Maleinsäuredi(meth)acryloyloxypropylester, Methylmaleinsäuredi(meth)acryloyloxypropylester, Bernsteinsäuredi(meth)acryloyloxypropylester, Glutarsäuredi(meth)acryloyloxypropylester, Adipinsäuredi(meth)acryloyloxypropylester, 1,2-Cyclohexandicarbonsäuredi(meth)acryloyloxypropylester oder 5-Norbornendicarbonsäuredi(meth)acryloyloxypropylester.

Um den Brechungsindex n_{D} noch weiter anzuheben, können als Verbindung (2) auch solche Verbindungen eingesetzt werden, deren Rest {B} zusätzlich mindestens eine aromatische Gruppe Ar trägt, die wie oben definiert ist. Beispiele sind Vinylbenzoesäure als Säure mit isolierter C=C-Doppelbindung, die Mono(meth)acryloyloxyethyl- oder -propylester der Phthalsäure, der 1,8-Naphthalsäure, der 4-Methacryloxyethyltrimellitsäure sowie die Mono- und Disäurechloride und -anhydride der vorgenannte difunktionellen Säuren sowie die Mono-, Di- und trisäurechloride und -anhydride der 4-Methacryloxyethyltrimellitsäure sowie Gemische der vorgenannten Verbindungen, insbesondere Gemische der jeweiligen Acryl- und Methacryl-Verbindungen.

Weitere Beispiele für Verbindungen (2) sind Hydroxyverbindungen mit isolierten C=C-Doppelbindungen wie Vinylalkohol, Allylalkohol, 1,5-Hexadien-3-ol, Diallylmethylcarbinol, 5-Norbornen-2-ol, Hydroxyverbindungen mit aktivierten C=C-Doppelbindungen wie 2-Hydroxyethyl(meth)acrylat, Glycerin-1,3-di(meth)acrylat, Oligoethylenglykolmono(meth)acrylate unterschiedlicher Kettenlänge, Pentaerythritoltri(meth)acrylat, N-(Hydroxymethyl)acrylamid, Gemische der vorgenannten Verbindungen, insbesondere Gemische der jeweiligen Acryl- und Methacryl-Verbindungen wie Glycerinacrylatmethacrylat.

Werden die Unterschüsse an Verbindungen (1) und (2) so gewählt, dass die Molmenge von (α + β) unter der Molmenge der cyclischen Ethergruppen liegt, entsteht ein Gemisch aus drei verschiedenen Silanen bzw. ein Kieselsäurepolykondensat, in welchem Reste (Ar)_{b}(W)ₐ-A-Y(R³)- mit R³ = OH neben Resten {B}-A"-Y(OH)- und entweder cyclische Ethergruppen (in Form des Ausgangssilans der Formel (III)) oder deren durch Ringöffnung entstandenes Reaktionsprodukt nebeneinander vorliegen. Dies soll nachstehend beispielhaft anhand der Umsetzung von 3-Glycidylpropylmethyldiethoxysilan mit α Mol Benzoesäure und β Mol Methacrylsäure gezeigt werden:

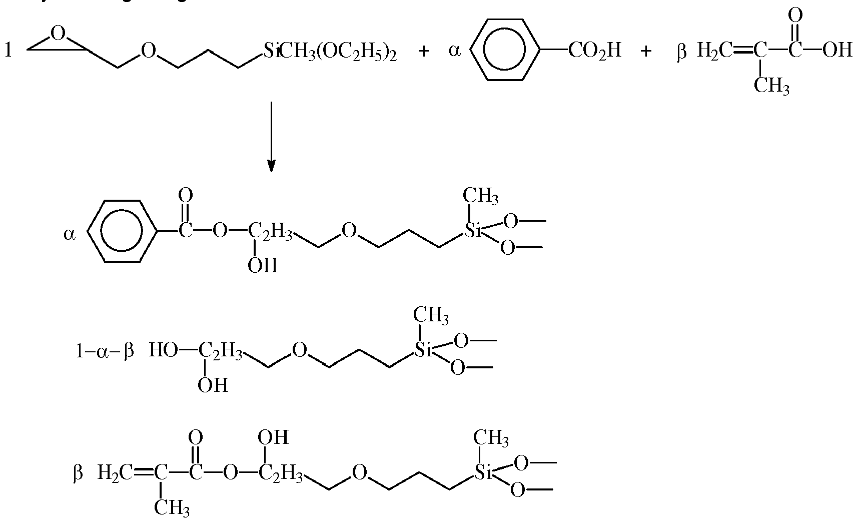

In Bezug auf das Entstehen der vicinalen Hydroxygruppen während der Hydrolyse bzw. auf die hydrolytische Kondensation unter Beibehaltung des geschlossenen cyclischen Ether-Rings gilt natürlich das zuvor zur Umsetzung mit nur der Verbindung (1) Gesagte.

Zwei oder drei der verschiedenen Substituenten können auch an ein und demselben Rest R¹ angebunden vorliegen, wenn z.B. anstelle eines Glycidyloxypropylrestes als R¹' ein solcher Rest eingesetzt wird, der zwei oder sogar noch mehr Glycidylgruppen trägt. Dann entstehen Systeme mit der Formel (I), in denen R¹ zusätzlich zum Substituenten Ar_{b}(W)ₐ-A-Y(OH)- mit {B}-A"-Y(OH)- substituiert ist und ggf. zusätzlich noch geschlossene cyclische Ethergruppen trägt.

Die oben dargestellte Reaktion mit der Verbindungen der Formeln (1) und (2) kann nicht nur gleichzeitig durchgeführt werden, was aus ökonomischen Gründen zu bevorzugen ist, sondern auch als sequenzielle Reaktion, indem das Silan der Formel (III) bzw. das entsprechende Kieselsäure(hetero)polykondensat zuerst mit der Verbindung (2) (im Unterschuss) und sodann mit der Verbindung (1) (nicht im Überschuss) umgesetzt wird. Auch die umgekehrte Sequenz ist möglich, wobei in diesem Fall die Verbindung (1) im Unterschuss und sodann die Verbindung (2) nicht im Überschuss eingesetzt wird. Würde die in der Sequenz jeweils als zweite und letzte eingesetzte Verbindung im Überschuss eingesetzt werden, könnte der überschüssige Anteil an den durch die Umsetzung mit der zuerst eingesetzten Verbindung gebildeten OH-Gruppen bzw. organisch polymerisierbaren C=C-Doppelbindungen angreifen. Letzteres kann jedoch gegebenenfalls sogar erwünscht sein, wie weiter unten dargestellt. Es ist aber festzuhalten, dass auch dann, wenn die Gruppen Q/Q' bevorzugt an den reaktiven cyclischen Ethergruppen angreifen, ihr Angriff an freie Hydroxygruppen bzw. an gegebenenfalls vorhandene C=C-Doppelbindungen und damit die Erzeugung von Nebenprodukten nicht in allen Fällen ausgeschlossen ist. Solche Nebenreaktionen treten aber dann nicht ein, wenn zuerst die Verbindung mit der Formel (1) und erst dann eine Verbindung mit der Formel (2) eingesetzt wird, wobei in letzterer Q' die Bedeutung eines freien Carbonsäurerestes -COOH oder von OH hat. Will man diese Nebenreaktionen also zuverlässig ausschließen, sollte für diese Umsetzung die Sequenz 1. Reaktion → 2. Reaktion gewählt und eine Verbindung {B}-Q' eingesetzt werden, in der Q' COOH oder OH ist.

Das Vorhandensein des oder der aromatischen Reste des Substituenten Ar_{b}(W)ₐ-A-Y(OH)-bewirkt eine Verschiebung, insbesondere eine Erhöhung des Brechungsindexes des Kieselsäure(hetero)polykondensats. Wie sich aus den vorstehenden Erläuterungen unschwer entnehmen lässt, kann die Zahl der aromatischen Reste dabei auf mehrfache Weise genau abgestuft werden. Dabei ist hervorzuheben, dass vor allem dann, wenn das Verhältnis von α zu β hoch ist oder die Umsetzung ausschließlich mit der Verbindung (1) erfolgt, eine hohe Anzahl an Substituenten Ar pro Silan-Molekül bzw. Si-Atomen im Kieselsäure(hetero)polykondensat und entsprechend eine hohe Dichte dieser Substituenten im Kieselsäure(hetero)polykondensat erzielt werden kann. Eine geringere Dichte gelingt entweder mit einer geringeren Anzahl von reaktiven cyclischen Ethergruppen oder durch deren Umsetzung mit einem Unterschuss an Verbindung (1). Daraus ergibt sich, dass die Erfindung in ihrer ersten Ausgestaltung (mit R³ = OH) Silan- und Kieselsäure(hetero)polykondensat-Systeme bereitstellen kann, in denen nicht nur (a) die Menge/Dichte an Substituenten Ar variabel einstellbar ist, sondern in denen (b) auch - durch entsprechende Wahl eines Unterschusses an Verbindung (1) - eine beliebig große Anzahl an zwei (meist vicinalen) Hydroxygruppen ausgebildet sein kann, während (c) weitere Hydroxygruppen durch die Umsetzung mit der Verbindung (1) ausgebildet werden, die stöchiometrisch im Verhältnis 1:1 zur Menge der Verbindung (1) entstehen, aber nicht notwendigerweise im Verhältnis 1:1, relativ zu den Substituenten Ar, vorhanden sein müssen. Letzteres liegt daran, dass die Substituenten Ar pro Molekül (1) b-fach vorhanden sind, während immer eine Hydroxygruppe pro Molekül Verbindung (1) entsteht. Wenn b 2 oder 3 bedeutet, entstehen nur 50 bzw. 33 Mol-% Hydroxygruppen pro Einführung von 100% an Substituent Ar auf diesem Wege. Daher kann die Relation von Hydroxygruppen zu Substituenten Ar von ca. 0,3:1 bis zu mehr als 3:1, gegebenenfalls (bei großem Unterschuss an Verbindung (1), relativ zu den reaktiven cyclischen Ethergruppen) 5:1 oder sogar bis zu 10:1 betragen. Es ist zu betonen, dass die Relation von Hydroxygruppen zu Substituenten Ar stufenlos und sehr fein einreguliert werden kann, wobei es eine einzige Reaktion ist, die diese Vielfalt ermöglicht.

Alle voranstehend beschriebenen Reaktionen führen zu Silanen oder Kieselsäure(hetero)polykondensaten mit Resten (Ar)_{b}(W)ₐ-A-Y(R³)-, in denen R³ = Hydroxy ist. Mit einem weiteren Reaktionsschritt lassen sich diese Hydroxygruppen kontrolliert in Reste D-{B} mit der in Formel (I) angegebenen Bedeutung überführen. In dieser weiteren Ausführungsform der Erfindung lässt sich diese Möglichkeit dazu nutzen, die Anzahl an organisch polymerisierbaren C=C-Doppelbindungen (nichtaromatischen C=C-Doppelbindungen) der erfindungsgemäßen Silane bzw. Kieselsäure(hetero)polykondensate nachträglich weiter zu erhöhen (wenn bereits Substituenten {B}-A"-Y(OH)- vorhanden sind), oder solche erstmals in die Systeme einzuführen, um eine organische Vernetzung der Systeme zu ermöglichen oder zu verbessern.

Erhältlich sind die Silane der Formel (I) bzw. die Kieselsäure(hetero)polykondensate, in denen Reste (Ar)_{b}(W)ₐ-A-Y(R³)- mit der Bedeutung R³ = D-{B} vorliegen, durch Umsetzung der oben dargestellten, entsprechenden Silane/Kieselsäure(hetero)polykondensate mit Gruppen der Formel

(Ar)_{b}(W)ₐ-A-Y(R³)- (II)

In denen R³ OH ist, mit einer Verbindung mit der Formel (3)

{B}-V (3),

worin V OH, NCO, eine gegebenenfalls alkyl- oder cycloalkylensubstituierte Epoxidgruppe oder COA' ist und A' Hydroxy, ein Halogenid oder -OC(O)R⁵ mit R⁵ gleich unsubstituierter oder substituierter Kohlenwasserstoff, z.B. Alkyl oder Alkylen bedeutet oder COA' ein cyclisches Anhydrid ist, das über zwei Kohlenstoffatome an {B} gebunden ist, und {B} die oben für Formel (II) angegebene Bedeutung besitzt. Dabei reagiert der Rest V mit den Gruppen R³ = OH unter Ausbildung der Kupplungsgruppe D, die dementsprechend ausgewählt sein kann unter einer Carboxylgruppe (Estergruppe), einer Ethergruppe, einer Urethangruppe und einer mit Hydroxy und ggf. (einem) weiteren Alkyl- oder Cycloalkylensubstituenten substituierten Ethylenoxygruppe. Die Umsetzung mit der Verbindung mit der Formel (3) wird als Reaktion 3 bezeichnet. Diese soll nachstehend anhand des Beispiels der Umsetzung eines Silans oder Kieselsäure(hetero)polykondensats mit einem Rest R¹, in dem R³ OH ist, mit einer Verbindung der Formel (3) gezeigt werden, worin V die Isocycanatgruppe OCN- bedeutet und der Rest {B} Methacrylsäureethylester ist, dessen Alkoholkomponente mit dem Isocyanatrest substituiert ist:

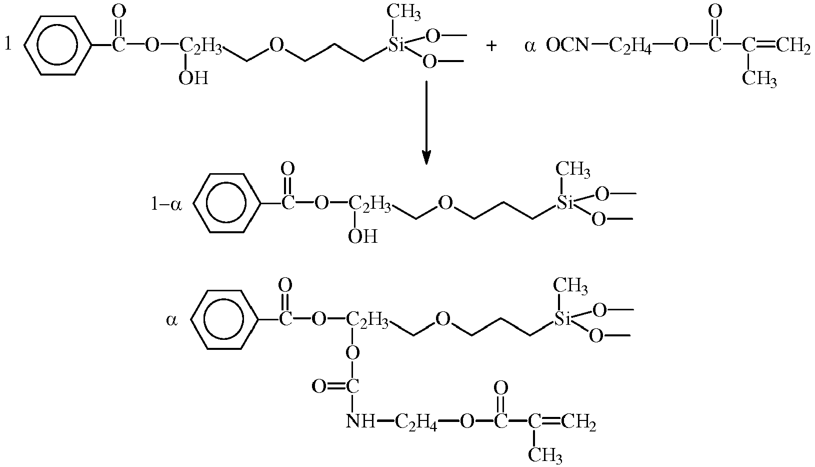

Wenn V unter Gruppen mit der Bedeutung COA' ausgewählt wird, greift der Rest V der Verbindung (3) gegebenenfalls außerdem verbliebene reaktive cyclische Ethergruppen oder daraus durch Ringöffnung entstandene Hydroxygruppen an und setzt auch diese um. Mit einer ausreichenden Mengen an Verbindung (3) erhält man ein erfindungsgemäßes System (Silan oder Kieselsäure(hetero)polykondensat), in dem alle zuvor vorhandenen Hydroxygruppen ersetzt sind durch D-{B}.

In einer Alternative zu dieser Reaktion kann die freie Hydroxygruppe R³ aber auch genutzt werden, um über eine Gruppierung -D-(W)ₐ(Ar)_{b} weitere Reste Ar in das System einzuführen, wobei D dieselbe Bedeutung wie zuvor für die Umsetzung mit der Verbindung (3) beschrieben besitzt. Dies gelingt, indem das erfindungsgemäße System mit Gruppen der Formel

(Ar)_{b}(W)ₐ-A-Y(R³)- (II)

In denen R³ OH ist, mit einer Verbindung der Formel (4)

(Ar)_{b}(W)ₐV (4)

umgesetzt wird, worin Ar, W, a und b wie oben für Formel (1) definiert sind und V OH, NCO, eine gegebenenfalls alkylsubstituierte Epoxidgruppe oder COA' ist und A' Hydroxy, ein Halogenid oder -OC(O)R⁵ mit R⁵ gleich unsubstituierter oder substituierter Kohlenwasserstoff, z.B. Alkyl oder Alkylen bedeutet. Wenn a = 1 ist, kann V auch eine cyclische Anhydridgruppe sein, die über zwei Kohlenstoffatome an W gebunden ist. Wenn a = 0 ist und b = 2 ist, kann V ein Anhydrid sein, dessen Kohlenstoffatome jeweils an einen Rest Ar gebunden sind. Diese Reaktion wird als Reaktion 4 bezeichnet.

Wie oben für die Reaktionen 1 und 2 dargestellt, ist es auch möglich, die Reaktionen 3 und 4 mit jeweils einem Unterschuss an Verbindung der Formel (3) bzw. (4) ablaufen zu lassen, und zwar sequentiell oder gleichzeitig. Dabei konkurrieren die Verbindungen der Formeln (3) und (4) um die freien Hydroxygruppen der Systeme. Dies soll nachstehend anhand der Umsetzung eines Silans oder Kieselsäure(hetero)polykondensats mit einem Rest R¹, in dem R³ OH ist, mit einer Verbindung der Formel (3), worin V die Isocycanatgruppe OCN- bedeutet und der Rest {B} Methacrylsäureethylester ist, dessen Alkoholkomponente mit dem Isocyanatrest substituiert ist, in Kombination mit einer Verbindung der Formel (4) gezeigt werden, worin V ebenfalls die Isocyanatgruppe bedeutet, a = 0 ist und Ar Phenyl bedeutet:

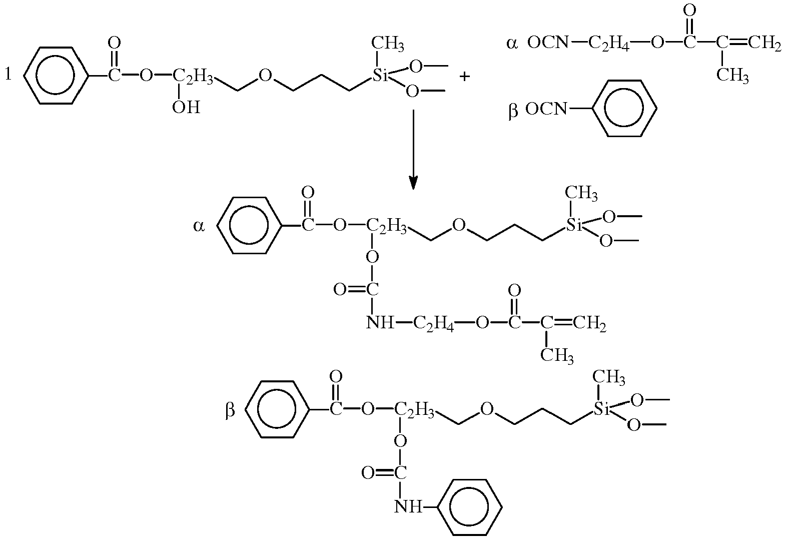

Verbindungen (1) sollten nie im Überschuss eingesetzt werden, wenn Q ein schwefel-, stickstoff- oder phosphorhaltiger Rest ist. Wenn in anderen Reaktionen 1 und/oder in Reaktionen 2 Überschüsse an Verbindungen (1) und/oder (2) eingesetzt werden, werden die bei der Ringöffnung des cyclischen Ethers entstehenden vicinalen Hydroxygruppen weiter umgesetzt. Man erhält Strukturen mit weiteren Resten (Ar)_{b}(W)ₐ-A bzw. {B}-A" an der Stelle einer der vicinalen Hydroxygruppen (es entsteht dabei ein Gemisch). In der Regel ist die Umsetzung mit einer zweiten Verbindung (1) und/oder (2) an benachbarten OH-Gruppe dann aus sterischen Gründen erschwert.

In einer weiteren Variante der Erfindung werden als Ausgangsmaterial Silane oder Kieselsäure(hetero)polykondensate eingesetzt, die zwei oder noch mehr reaktive cyclische Ethergruppen an einem über Kohlenstoff am Silicium gebundenen Rest tragen. Werden diese mit einer molaren Menge an Verbindung (1) umgesetzt, die nur eine dieser cyclischen Ethergruppen in die Gruppe (II) mit R³ = OH umwandelt, steht im Durchschnitt eine weitere (oder sogar noch mehr) cyclische Ethergruppe zur Anbindung einer Verbindung (2) zur Verfügung. In dieser Variante entstehen Silane oder Kieselsäure(hetero)polykondensate, in denen ein Rest R¹ neben der Gruppe (II) mit einer Gruppe der Formel (V)

{B}-A"-Y(OH)- (V)

substituiert ist, worin {B} und Y wie voranstehend definiert sind und A" ausgewählt ist unter -C(O)O- und -O-. Da die Verbindung (1) nicht stöchiometrisch genau jeweils an nur einer cyclischen Ethergruppe pro Rest R¹ angreift, entstehen (statistisch verteilte) Gemische.

Die erfindungsgemäßen Kieselsäure(hetero)polykondensate können entweder ausschließlich aus den oben beschriebenen Silanen aufgebaut sein; an ihrem Aufbau können aber auch andere, bekannte Silane beteiligt sein, die z.B. nichtaromatische C=C-Doppelbindungen enthalten. Beispiele für derartige Silane lassen sich den folgenden Druckschriften entnehmen: DE 40 11 044 A1, EP 450 624, DE 44 16 857 C1, DE 196 27198, DE199 10 895 A1, DE 103 49 766 A1, DE 101 32 654 A1, DE 10 2005 018 059 A1, DE 10 2011 054 440 A1, DE 10 2011 053 865 A1 und DE 10 2012 109 685 A1. Neben Silanen mit einem geradkettigen oder verzweigten organischen Rest, der eine oder zwei C=C-Doppelbindungen und 5 bis 50 Kohlenstoffatomen trägt und über eine Gruppierung A mit A = O, S, PR", POR" oder NHC(O)O an den über Kohlenstoff an das Silicium gebundenen Rest verknüpft ist wie in DE 40 11 044 A1 offenbart oder worin die genannte Verknüpfung über eine Säureamid-Gruppe erfolgt wie in DE 199 10 895 A1 offenbart, können das beispielsweise Silane sein, die ähnlich aufgebaut sind wie die vorgenannten, jedoch zusätzlich eine Hydroxy- oder COOH-Gruppe wie in DE 44 16 857 C1 offenbart oder eine Phosphor enthaltende Gruppe, beispielsweise eine Phosphonsäure aufweisen wie aus DE 101 32 654 A1 bekannt. Andere Beispiele sind in DE 103 49 766 genannt; diese Silane weisen mindestens zwei gleiche oder unterschiedliche C=C-doppelbindungshaltige Reste auf, die in unterschiedlichen Abständen zum Siliciumatom am über Kohlenstoff gebundenen Kohlenwasserstoffrest angebunden sind, wobei mindestens eine der C=C-doppelbindunghaltigen Reste über eine Kopplungsgruppe -NH-C(O)O-, -NH-C(O)- oder -CO(O)- mit dem Kohlenwasserstoffrest verknüpft ist. Und schließlich sei auf Silane verwiesen, die mindestens eine Norbornenyl- oder verwandte Gruppierung enthalten, wie in DE 106 27 198 A1 erläutert.

Ein besonderer Vorzug der vorliegenden Erfindung liegt also darin, dass sich bei geeigneter Wahl eines Ausgangsmaterials, das ja aus dem Stand der Technik zur Verfügung steht, mit nur einer Umsetzung beliebige Abstufungen erzielen lassen, denn mit der Wahl des Ausgangsmaterials lässt sich bereits der Anteil an Gruppen R¹ pro Siliciumatom vorgeben. Mit der Wahl des stöchiometrischen Verhältnisses von Verbindung (II) zu Rest R³ sowie der Menge an Gruppen Ar pro Verbindung (II) lässt sich dann die Relation von Siliciumatomen zur Anzahl der Gruppen Ar, die Relation von Siliciumatomen zur Anzahl der verbliebenen Reste R¹ sowie die Relation der Anzahl der Gruppen Ar zur Anzahl der verbliebenen Reste R¹ völlig frei festlegen. Dabei hat die Umsetzung auf der Ebene der Kieselsäurepolykondensate den Vorteil, dass man ausgehend von einem einzigen Kondensat und mit nur jeweils einem Schritt pro hergestelltem Kondensat zu einer fein abgestuften Produktpalette gelangen kann.

Wie bereits weiter oben kurz erwähnt, kommt es bei der Umsetzung von (III) mit einem Unterschuss an (1) zu Struktur (I) unter Vermeidung hydrolytischer Bedingungen zur Entstehung eines Gemischs, das verbliebene, noch geschlossene cyclische Epoxidgruppen aufweist. Diese Epoxidgruppen können im Rahmen einer Epoxidpolymerisation zum alleinigen Aufbau einer organischen Polymerstruktur genutzt werden. Dies gilt auch für den Fall, dass (III) sowohl mit einer Verbindung der Formel (1) als auch mit einer Verbindung der Formel (2) umgesetzt wird, sofern die molare Summe beider Verbindungen immer noch einen Unterschuss relativ zu (III) ergibt. Im letzteren Fall besitzen die Strukturen (I) sowohl reaktive cyclische Epoxidgruppen als auch organisch polymerisierbare C=C-Doppelbindungen. Mit diesen Strukturen lassen sich zwei unterschiedliche, durch gleiche Initiierung (z. B. beide photoinitiiert) bzw. unterschiedliche Initiierung (z. B. einmal photo- und einmal thermisch initiiert und damit ein Aufbau der beiden Netzwerke in unterschiedlicher Reihenfolge), erhaltene Netzwerkstrukturen in variablen Anteilen erzeugen.

Die Ringstrukturen wie die Epoxidgruppen können unter üblichen, allgemein bekannten Bedingungen kationisch ringöffnend polymerisiert werden (thermisch bzw. photoinitiiert nach Zugabe geeigneter Initiatoren wie Lewis- bzw. Brönsted-Säuren (wie BF₃, BF₃·THF, BF₃·Et₂O, AlCl₃, HPF₆, HBF₄) bzw. Verbindungen die solche Säuren freisetzen). Bei der Homopolymerisation entstehen so Polyetherstrukturen. Beispielhafte Photoinitiatoren sind Rhodorsil®2074 (ein Diaryljodoniumborat) bzw. Degacure®KI85 (Bis[4-(diphenylsulfonio)-phenyl]sulfid-bis-hexafluorophosphat). Weiterhin können die Ringstrukturen durch di-, tri- bzw. tetrafunktionelle Verbindungen wie z. B. Di-, Tri- bzw. Tetraamine, Di-, Tri- bzw. Tetrathiole, Di-, Tri- bzw. Tetraanhydride, Di-, Tri- bzw. Tetracarbonsäuren vernetzt und somit ausgehärtet werden. Die entsprechenden Synthesen entsprechen den voranstehend im Zusammenhang mit den Additionsreaktionen an die Epoxidgruppen der erfindungsgemäßen Systeme erläuterten Reaktionen. Da die mehrfunktionellen Verbindungen an mehreren Ringstrukturen angreifen können, entstehen rein organische Brücken zwischen den entsprechenden Gruppen.

Bei den nichtaromatischen C=C-Doppelbindungen des Restes {B} handelt es sich um Gruppierungen, die unter dem Einfluss von Wärme, Licht, ionisierender Strahlung oder redoxinduziert (z.B. mit einem Initiator (Peroxid oder dgl.) und einem Aktivator (Amin oder dgl.)) einer organischen Vernetzung unterworfen werden und dabei in Polymere übergehen (engl.: addition polymerization oder chain-growth polymerization) können. Unter dem Attribut "polymerisierbar" bzw. dem entsprechenden Substantiv "Polymerisation" ist daher vorliegend eine entsprechende Polyreaktion zu verstehen, bei der weder Abspaltungen von molekularen Bestandteilen auftreten, noch Wanderungen oder Umlagerungen. Beispiele für {B} sind einer Michaeladdition zugängliche Doppelbindungen wie Styryle oder (Meth)Acrylsäure-Derivate; es kann sich aber auch um Vinyl- oder Allylgruppen handeln. Alle Silane und Kieselsäure(hetero)-polykondensate der vorliegenden Erfindung, die Gruppen {B} tragen, können auf diese Weise vernetzt werden, wobei eine Vernetzung von Silanen vorzugsweise erst nach deren Polykondensation erfolgt. Eine solche Polymerisationsreaktion macht eine z.B. lichtinduzierte nachträgliche Vernetzung des Kieselsäurepolykondensats möglich, mit der beispielsweise im Rahmen einer Dentalapplikation (z.B. bei Einsatz eines Zahn-Restaurationsmaterials) das eingesetzte Kieselsäure(hetero)polykondensat bzw. ein Komposit aus diesem Kondensat als Matrix und darin eingebetteten Füllpartikeln nach Abschluss der Formgebung im Mund des Patienten gehärtet werden kann. Es gibt jedoch auch noch andere Wege, um eine nachträgliche organische Vernetzung des erfindungsgemäßen Kieselsäurepolykondensats zu ermöglichen, was nachstehend dargestellt wird.

Diese spezielle Form der Nachhärtung nutzt nicht (oder nicht nur) die Polymerisationsreaktion (Polyaddition) der C=C-Doppelbindungen als solcher wie oben erläutert. Möglich ist nämlich auch eine Umsetzung der diese Doppelbindungen enthaltenden Silane bzw. Kieselsäurepolykondensate mit Di- oder höheren Aminen oder Di- oder höheren Thiolen über eine Michael-Addition (Thiol-En-Umsetzung bzw. die analoge Umsetzung mit Aminen). Möglich ist dies mit Di-, Tri, Tetra- oder sogar noch höher funktionalisierten Aminen oder Mercaptanen, wobei die Reaktion mit Aminen (nur) dann gelingt, wenn die C=C-Doppelbindungen in aktivierter Form vorliegen, beispielsweise als Acryl- oder Methacrylgruppen (darunter (Meth)acrylatgruppen). Hier kann an die Stelle der Vernetzung durch lichtinduzierte organische Polyaddition ("chain growth polymerization") wie oben erwähnt die Vernetzung durch die genannten, mehrfach funktionellen Mercaptane bzw. Amine treten.

Beispiele für polyfunktionelle, zur Verbrückung sowohl über die Ringöffnung von reaktiven cyclischen Ethern als auch über die Umsetzung mit C=C-Doppelbindungen einsetzbare Verbindungen sind die folgenden:
Beispiele für multifunktionellen Thiole: Trimethylolpropantri-(3-mercaptopropionat) (TMPMP); Trimethylolpropan-trimercaptoacetat) (TMPMA); Pentaerytritoltetra(3-mercaptopropionat) (PETMP); Pentaerytritoltetramercaptoacetat) (PETMA); Glykoldimercaptoacetat; Glykoldi(3-mercapto-propionat); Ethoxyliertes Trimethylolpropantri(3-mercaptopropionat); Biphenyl-4-4'-dithiol; p-Terphenyl-4,4"-dithiol; 4,4'-Thiobisbezenthiol; 4,4'-Dimercaptostilben; Benzen-1,3-dithiol; Benzen-1,2-dithiol; Benzen-1,4-dithiol; 1,2-Benzendimethanthiol; 1,3-Benzen-dimethanthiol; 1,4-Benzendimethanthiol; 2,2'-(Ethylendioxy)diethanthiol; 1,6-Hexandithiol; 1,8-Octandithiol; 1,9-Nonandithiol. Gleiches gelingt, wenn statt der oben beschriebenen Thiole Amine verwendet werden, sofern, wie erwähnt, die C=C-Doppelbindungen in aktivierter Form vorliegen.

Beispiele für multifunktionelle Amine: Diaminoaceton, Diaminoacridin, Diaminoadamantan, Diaminoanthrachinon, Benzidin, Diaminobenzoesäure, Phenylendiamin, Diaminobenzophenon, Diaminobutan, Diaminocyclohexan, Diaminodecan, Diaminodicyclohexylmethan, Diaminomethoxybiphenyl, Diaminodimethylhexan, Diaminodiphenylmethan, Diaminododecan, Diaminoheptan, Diaminomesitylen, Diaminomethylpentan, Diaminomethylpropan, Naphtyhlendiamin, Diaminoneopentan, Diaminooctan, Diaminopentan, Diaminophenanthren, Diaminopropan, Diaminopropanol, Diaminopurin, Diaminopyrimidin. In der Regel wird die Thiol-Addition in Gegenwart eines Initiators durchgeführt, wie aus dem Stand der Technik bekannt, während die Amin-Addition auch ohne Initiator möglich ist.

Die genannte Nachhärtung (Polyaddition) kann an die Stelle der Polymerisationsreaktion der C=C-Doppelbindungen (der "chain growth polymerization") treten; es bildet sich dabei ein etwas lockereres organisches Netzwerk, weil S-(Kohlenwasserstoff)-S-Brücken bzw. N-(Kohlenwasserstoff)-N-Brücken ausgebildet werden. Sie kann aber auch zusätzlich erfolgen, indem die Menge an Di- bzw. Polythiolen oder Di- bzw. Polyaminen so gewählt wird, dass C=C-Doppelbindungen im Harz verbleiben, die anschließend auf üblichem Wege nachgehärtet werden können.

Eine weitere Vernetzungsmöglichkeit ist über eine Variante der Reaktionen 1 und 2 gegeben, sofern das/die Produkte der ersten Umsetzung immer noch reaktive cyclische Ethergruppen aufweisen: Werden neben den Verbindungen (1) und/oder (2) zusätzlich Verbindungen (1')

(Ar)_{b}(W)ₐ-[Q]_{c} (1')

und/oder (2')

{B}-[Q']_{c} (2')

eingesetzt, in denen c = 2 oder größer ist, entstehen über die Reaktion von (1') und/oder (2') mit den cyclischen Ethergruppen Verbrückungen zwischen zwei Resten Y. Mit dieser Vernetzung können Eigenschaften wie die Länge der Molekülketten zwischen den Vernetzungsstellen sowie die verbleibenden Anteile an freien reaktiven Gruppen eingestellt werden. Beispielsweise kann ein variabler Anteil an Di-, Tri- und/oder Tetra-Isocyanaten als Verbindung (1') eingesetzt werden, wobei die Isocyanatgruppen mit jeweils freien OH-Gruppen reagieren können. Durch den Einsatz bis-, tris- oder noch höher funktioneller Verbindungen als Reaktionspartner mit der Formel (1') oder (2') werden Verknüpfungen über Brücken mit wahlweise einstellbarer Länge erzeugt (die Längeneinstellung erfolgt durch den Abstand der reaktiven Gruppen Q im Molekül). Als Beispiel hierfür diene die Umsetzung mit Isocyanaten: Hier können z.B. Dicyclohexylmethandiisocyanat, Hexamethylen-1,6-diisocyanat, Hexamethylen-1,8-diisocyanat, Diphenylmethan-4,4-diisocyanat, Diphenylmethan-2,4-diisocyanat, 2,4-Toluylendiisocyanat, 2,6-Toluylendiisocyanat, Triphenylmethan-4,4',4"-triisocyanat (ein weiterer Vorteil der Umsetzung mit den letztgenannten Verbindungen liegt aufgrund des Vorhandenseins aromatischer Gruppe in der nochmaligen Erhöhung der Brechzahl), 3-Isocyanatomethyl-3,5,5-trimethylcyclohexyl-diisocyanat, oder Tris(p-isocyanatophenyl)thiophosphat eingesetzt werden. Sofern es sich bei den Resten R² um eine Hydroxygruppe handelt, kann eine derartige Vernetzung z.B. auch mit einer di-, tri-, tetra oder polyfunktionellen, ggf. aktivierten (z.B. in Form eines Anhydrids vorliegenden) Carbonsäure anstelle des Di-, Tri-, Tetra oder noch höheren Polyisocyanats erfolgen, während dann, wenn es sich bei diesem Rest um einen freien Carbonsäurerest, oder dessen Salz oder Ester handelt, aufweist, die Vernetzung statt dessen auch mit einem di-, tri-, tetra oder polyfunktionellen Alkohol oder Epoxid erfolgen kann.

Die erfindungsgemäßen Harze werden für dentale Zwecke im Allgemeinen in mit Partikeln gefüllter Form eingesetzt. Geeignete Füllstoffe sind beispielsweise diejenigen, die in der DE 196 43781, DE 19832965, DE 10018405, DE 1041038, DE102005018351, DE102005061965.7 und insbesondere in der DE102011054444.5 beschrieben sind. Bei diesen Füllstoffen handelt es sich um Beispiele für prinzipiell mögliche Füllstoffe zur Kompositherstellung. Bevorzugt ist allerdings, dass klassische, kommerziell erhältliche Dentalglasfüller z. B. der Fa. Schott, zumindest einen wesentlich Anteil des Füllstoffs (vorzugsweise über 50 Gew.-%, stärker bevorzugt über 75 Gew.-%) bilden. In diesen Ausführungsformen kann der Rest z.B. aus Füllstoffen, wie sie in den oben genannten Druckschriften genannt sind, bestehen. Durch Rückgriff auf die bevorzugten Dentalglasfüller, ggf. in Kombination mit weiteren Füllstoffen wie voranstehend dargestellt, ist es möglich, die Brechzahl der erfindungsgemäßen Harzsysteme und diejenige des Füllstoffs/Dentalglases soweit aneinander anzupassen, dass sie fast (oder, vorzugsweise, sogar vollständig) identisch sind. Es lassen sich Brechzahldifferenzen von Δ_{nD} im Bereich von ± 0,001, häufig von ± 0,0005 erreichen. Auf diesem Wege erhält man die gewünschte, auch notwendige Transluzenz der erfindungsgemäßen Komposite, die sich damit als hochästhetische dentale Werkstoffe einsetzen lassen.

Sofern die voranstehenden Umsetzungen an Silanverbindungen erfolgen, können diese, wie bereits erwähnt, anschließend einer hydrolytischen Kondensation unterworfen werden. Eine derartige Kondensationsreaktion kann entweder an einer einzigen erfindungsgemäßen Silanverbindung oder an einer Mischung aus mehreren Silanverbindungen erfolgen, die durch eine oder mehrere der voranstehend beschriebenen Reaktionen entstanden sind. In allen Fällen können je nach Bedarf weitere, bekannte Silane oder Alkoxymetallverbindungen wie Alkoxide des Aluminiums, Titans, Bors oder Zirkoniums oder von weiteren Übergangsmetallen einkondensiert werden. Durch den Zusatz von Silanen mit vier hydrolysierbaren Gruppen wie Tetraalkoxysilanen und/oder Alkoxymetallverbindungen kann beispielsweise das anorganische Netzwerk gefestigt und die Anzahl der organischen Substituenten "verdünnt" werden, während man durch den Zusatz von Silanen mit zwei oder drei derartigen hydrolysierbaren Gruppen und einem oder zwei über Kohlenstoff an Silicium gebundenen Substituenten verschiedenartige Modifikationen vornehmen kann. So modifiziert eine höhere Anzahl von silangebundenen Alkylgruppen die mechanischen Eigenschaften des letztendlich ausgehärteten Kieselsäurepolykondensats, während silangebundene Kohlenwasserstoffgruppen mit reaktiven Substituenten die Möglichkeit zusätzlicher Reaktionen ermöglichen. Silangebundene Kohlenwasserstoffgruppen, die organisch polymerisierbare C=C-Doppelbindungen enthalten, können zugesetzt werden, um die organische Vernetzungsmöglichkeit für den Fall zu gewährleisten, dass unter spezifischen Umständen die Umsetzungen des Silans bzw. der Struktur (1) mit Verbindungen der Formel (II) und/oder (III) hierfür entweder nicht ausreichen oder aus anderen Gründen nicht durchgeführt werden sollen.

Mit den in der vorliegenden Anmeldung beschriebenen neuartigen funktionalisierten Harz-/ Matrix-Systemen stehen Materialien zur Verfügung, die einerseits aufgrund ihrer besonderen Eigenschaften (feinjustierbare Brechzahl bei anpassbarem E-Modul, ggf. matrixinterne Stabilisierung durch Thiobrücken) direkt verwendet werden können und sich daher z.B. aufgrund der durch die Einführung der Arylgruppen erzielten Brechzahlsteigerung für besondere optische Anwendungen eignen können, andererseits aber gerade aufgrund der feinen Justierbarkeit der Brechzahl mit Füllstoffen andererseits zu beispielsweise im dentalen Bereich anwendbaren Kompositen verarbeiten lassen, bei denen je nach Bedarf die Brechzahlen des Harzes und des Füllstoffes annähernd oder ganz identisch gewählt werden können, so dass eine hohe Transluzenz erreicht wird. Die Harze zeichnen sich durch die Möglichkeit aus, plastisch verarbeitbare Komposite mit sehr hohen Füllstoffgehalten (bis über 85 Masse-%, in Einzelfällen sogar bis über 89 Masse%, und damit über 80 Vol.-%) zu erzeugen. In gehärteter Form zeigen sie einen an die jeweilige Anwendung anpassbaren E-Modul bei hoher Festigkeit, eine hohe Abrasionsresistenz, eine sehr geringe Schrumpfung, eine ausgezeichnete Ästhetik (anpassbare Transluzenz bis zu vollständiger Transluzenz), Monomerfreiheit und damit eine hohe Biokompatibilität. Außerdem konnten die Erfinder den überraschenden Effekt beobachten, dass in solchen Fällen, in denen mit einer Thiolverbindung als Verbindung der Formel (1) gearbeitet wird, eine matrixinterne Stabilisierung durch die Thiobrücke eintritt, z.B. gegenüber der bei höheren Temperaturen oftmals bei klassischen Polymeren beobachteten, oxidativen Zersetzung durch Radikalreaktionen.

Der Einsatz der erfindungsgemäßen Materialien ist vielgestaltig; er erstreckt sich zum Beispiel für die verschiedensten Zwecke auf die Verwendung in Form von Bulkmaterialien, Kompositen, Zementen, Klebstoffen, Vergussmassen, Beschichtungsmaterialien, Haftvermittlern, zur Herstellung bzw. Primung von Füllstoffen und Fasern, und zur Verarbeitung im (Reaktions)Extruder. Insbesondere ist ein Einsatz für (zahn-)medizinische und für (mikro)optische und (mikro)elektronische Anwendungen von Bedeutung.

Besonders bevorzugt ist der Einsatz im
▪ Dentalbereich, z. B. als Restaurations-/Zahnersatz (Kronen, Brücken, Inlays, Onlays, Veneers, künstliche Zähne, Implantataufbauten, Abutments,) oder Prophylaxematerial, Adhäsiv, Ionomerzement, in der Prothetik, als Implantatmaterial,
▪ Bereich der Mehrphotonenpolymerisation (sofern das fertige Material organisch polymerisierbare C=C-Doppelbindungen aufweist): Das flüssige Harz kann in einem Bad mit fokussierten Laserstrahlen behandelt werden, derart, dass nur im Fokus der Laserstrahlung eine Verfestigung durch die Polymerisation der C=C-Gruppen in den Resten R¹ auftritt, wodurch sich innerhalb des Bades ein Festkörper mit beliebigen Formen und genauen Abmessungen erzeugen lässt. Dieser Einsatz ist dem Bereich der Optik zuzuordnen.

Die vorliegende Erfindung besitzt weiterhin die folgenden Vorteile:
▪ Es handelt sich bei den beschriebenen Additionsreaktionen in der Regel um sehr schnelle und einfache Umsetzungen unter milden Bedingungen (z. T. ohne Katalysator bzw. mit sehr geringer Katalysator-Konzentrationen) ohne zusätzliche Aufarbeitungs-/ Reinigungsschritte, die zu den höher brechenden Strukturen (infolge des Vorhandenseins von Arylresten), den Strukturen mit erhöhtem Vernetzungspotenzial bei erhöhter Brechzahl (infolge des Vorhandenseins von Arylresten sowie organisch polymerisierbaren C=C-Doppelbindungen) bzw. den vernetzten Strukturen mit ggf. weiter erhöhter Brechzahl (Einführung weiterer Arylreste über die Gruppe R³ zu D-(W)-(Ar)_{b}) führen.
▪ Die Erfindung ermöglicht ausgehend von bereits existierenden Silanen und Harzsystemen durch deren nahezu stufenlose Modifikationsmöglichkeiten wie voranstehend dargestellt, insbesondere infolge der feinen Justierbarkeit von Brechzahl und E-Modul wie oben erwähnt eine Brechzahlanpassung an z. B. beliebige kommerziell erhältliche Füllstoffe, weshalb die Notwendigkeit, zur Erzeugung einer gewünschten, meist extrem hohen Transluzenz auf besonders angepasste und damit teure Füllstoffe zurückgreifen zu müssen, wegfällt. Damit ist einerseits im Dentalbereich die geforderte Ästhetik von dentalen direkten/indirekten Restaurations-/Zahnersatzmaterialien realisierbar, während andererseits das Erzielen einer vollständigen Transparenz optische Anwendungen ermöglicht. Darüber hinaus lassen sich, wie oben erwähnt, die mechanischen Eigenschaften, z.B. der E-Modul unabhängig von den optischen Eigenschaften auf die jeweils erforderlichen Werte einstellen.
▪ Für die Anwendung von Materialien im Dentalbereich ist es wichtig, dass diese keine allergischen Reaktionen auslösen können. Es ist bekannt, dass einige (meth)acrylat-basierte Monomere aus toxokologischer Sicht problematisch sind, weshalb für die genannten Anwendungen in der Regel auf diese verzichtet werden muss. Sofern jedoch die Materialien der vorliegenden Erfindung (Meth)Acrylgruppen aufweisen, beispielsweise in Form der Reste R¹ oder als Bestandteil von Resten R¹, besteht keinerlei Gefährdung, da sie im Harz in jedem Fall mit der anorganischen Polymerstruktur (basierend auf der anorganischen Vernetzung über Si-O-Si-Brücken) verbunden sind und somit weder im nicht ausgehärteten Zustand (in dem die Harze möglicherweise dental appliziert werden) noch nach der Härtung (z.B. durch lichtinduzierte Vernetzung der C=C-Doppelbindungen im Mund des Patienten) in monomerer Form vorhanden sein und aus dem Dentalkörper austreten können. Im Übrigen sei darauf hingewiesen, dass diese Überlegungen keineswegs ausschließen, dass den erfindungsgemäßen Silanen, Kieselsäure(hetero)polykondensaten und Kompositen bei Bedarf organische Monomere zugesetzt werden können.
▪ Von besonderer Bedeutung ist die sehr einfache, nahezu stufenlose Modifikationsmöglichkeit und damit feine Justierbarkeit der erfindungsgemäßen Kieselsäure(hetero)polykondensate bzw. den daraus erzeugbaren Kompositen, wobei die Erfindung von bereits im Stand der Technik vorhandenen Harzsystemen (ggf. statt dessen Silanen) ausgeht und dem Fachmann eine Auswahl geeigneter Materialien aus dieser Gruppe an die Hand gibt. Die erste bzw. einzige Umsetzung erfolgt mit einem meist chemisch einfach aufgebauten und daher in der Regel kostengünstigen Reaktionspartner; die Reaktionsbedingungen können milde gewählt werden, und zusätzliche Aufarbeitungs- oder Reinigungsschritte sind nur in Ausnahmefällen erforderlich.

Nachfolgend soll die Erfindung anhand von Ausführungsbeispielen näher erläutert werden.

### Vergleichsharzsystem 1 (Synthese gemäß DE 44 16 857 C1)

Zur Vorlage von 125,0 g (0,503 mol) 3-Glycidyloxypropylmethyldiethoxysilan werden unter trockener Atmosphäre Triphenylphosphin als Katalysator, BHT (3,5-Di-tert.-butyl-4-hydroxytoluol) -als Stabilisator und anschließend 47,35 g (0,550 mol) Methacrylsäure zugetropft und bei 80 °C gerührt (ca. 24 h). Die Umsetzung kann über die Abnahme der Carbonsäurekonzentration mittels Säuretitration sowie dem Epoxidumsatz mittels Epoxidtitration/NMR verfolgt werden. Die für die Epoxidgruppe vom Epoxysilan. Nach Zugabe von Essigester (1000 ml/mol Silan) und H₂O zur Hydrolyse mit HCl als Kat. wird bei 30 °C gerührt. Die Aufarbeitung erfolgt nach mehrtägigem Rühren durch Ausschütteln mit wäßriger NaOH und Wasser und Filtration über hydrophobierten Filter. Es wird zunächst abrotiert und anschließend mit Ölpumpenvakuum abgezogen. Es resultiert ein flüssiges Harz mit einer Viskosität von ≈ 3 - 5 Pa·s bei 25 °C sowie einer Brechzahl n_{D} ≈ 1,477.

### Beispiel 1 Grundharzsynthese 1 (Herstellung eines Kieselsäurepolysiloxans mit R³ = OH)

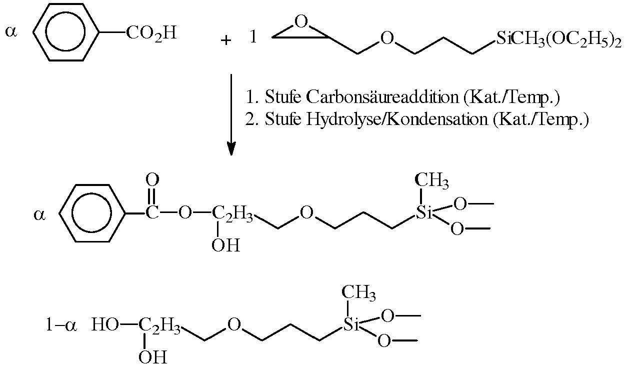

(im Kieselsäurepolykondensat sind die beiden gezeigten Produkt-Strukturen in das Siloxan-Netzwerk eingebunden)

### Beispiel 1a (Eduktanteile: α = 1):

Zur Vorlage von 49,7 g (0,20 mol) 3-Glycidyloxypropylmethyldiethoxysilan werden 0,26 g Triphenylphosphin als Katalysator und anschließend 24,5 g (0,20 mol) Benzoesäure zugegeben und bei ≈ 85 °C gerührt (ca. 24 h). Die Umsetzung kann über die Abnahme der Carbonsäurekonzentration mittels Säuretitration sowie dem Epoxidumsatz mittels Epoxidtitration/NMR verfolgt werden. Nach Zugabe von Essigester (1000 ml/mol Silan) und H₂O zur Hydrolyse mit HCl als Kat. wird bei 30 °C gerührt. Der Verlauf der Hydrolyse wird durch Wassertitration verfolgt. Die Aufarbeitung erfolgt nach mehrtägigem Rühren durch Ausschütteln mit wäßriger NaOH und Wasser und Filtration über hydrophobierte Filter. Es wird zunächst abrotiert und anschließend mit Ölpumpenvakuum abgezogen. Es resultiert ein flüssiges Harz ohne Einsatz von Reaktivver-dünneren (Monomeren) mit einer Viskosität von ca. 57 - 73 Pa·s bei 25 °C sowie einer Brechzahl n_{D} ≈ 1,521.

Weitere Aspekte:
▪ Brechzahl fein einstellbar über den Benzoesäureanteil (gegenüber dem Vergleichsharzsystem-1 und Beispiel 3 steigt die Brechzahl von > 1,477 bis ≈ 1,521)

### Beispiel 1b (Eduktanteile: α = 0,6):

Zur Vorlage von 50,18 g (0,202 mol) 3-Glycidyloxypropyltrimethoxysilan werden 0,52 g Triphenylphosphin als Katalysator und anschließend 14,67 g (0,12 mol) Benzoesäure zugegeben und bei ≈ 85 °C gerührt (ca. 4 h). Die Umsetzung kann über die Abnahme der Carbonsäurekonzentration mittels Säuretitration sowie dem Epoxidumsatz mittels Epoxidtitration/NMR verfolgt werden. Das resultierende flüssige Produkt kann direkt zur weiteren Umsetzung (Hydrolyse/Kondensation und weitere Additionen) eingesetzt werden.

### Beispiel 2 Grundharzsynthese 2 (Herstellung eines Kieselsäurepolysiloxans mit R³ = OH)

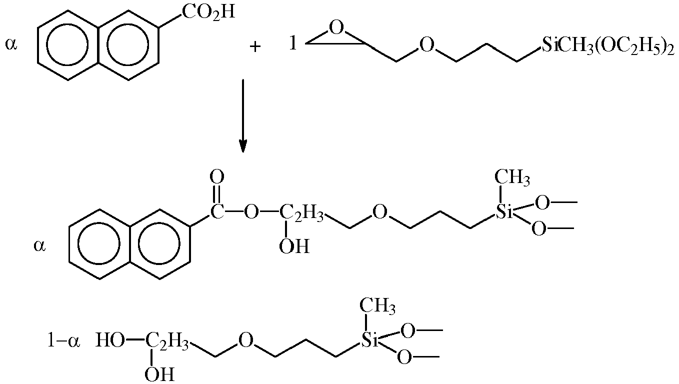

### Beispiel 2a

Das Verfahren des Beispiels 1a wurde wiederholt, wobei jedoch anstelle von Benzoesäure die entsprechende molare Menge an Naphthoesäure eingesetzt wurde.

### Beispiel 2b

Das Verfahren des Beispiels 1b wurde wiederholt, wobei jedoch anstelle von Benzoesäure die entsprechende molare Menge an Naphthoesäure eingesetzt wurde.

### Beispiel 3 gleichzeitige Umsetzung mit Verbindung (1) und (2)

Umsetzung eines Epoxidsilans mit Naphthoesäure und Methacrylsäure (das Produkt entspricht dem in Anspruch 12 beanspruchten Kieselsäurepolykondensat; auf der Ebene der Silane entsteht eine Mischung wie in Anspruch 5 beansprucht).

### (Eduktanteile: α = 0,5 (Naphthoesäure); β = 0,55 (Methacrylsäure)):

Zur Vorlage von 74,7 g (0,30 mol) 3-Glycidyloxypropylmethyldiethoxysilan werden 0,39 g Triphenyl-phosphin als Katalysator, 17,2 g (0,10 mol) Naphthoesäure und anschließend 9,47 g (0,10 mol) Methacrylsäure und bei ≈ 85 °C gerührt (ca. 2 Tg). Die Umsetzung kann entsprechend Beispiel 1 verfolgt werden. Nach Zugabe von Essigester (1000 ml/mol Silan) und H₂O zur Hydrolyse mit HCl als Kat. wird bei 30 °C gerührt. Der Verlauf der Hydrolyse wird durch Wassertitration verfolgt. Die Aufarbeitung erfolgt nach mehrtägigem Rühren durch Ausschütteln mit wäßriger NaOH und Wasser und Filtration über hydrophobierte Filter. Es wird zunächst abrotiert und dann mit Ölpumpenvakuum abgezogen. Es resultiert ein flüssiges Harz ohne Einsatz von Reaktivverdünnern (Monomeren) mit einer Viskosität von ca. 190 Pa·s bei 25 °C sowie einer Brechzahl n_{D} ≈ 1,535.

Weitere Aspekte:
▪ Brechzahl fein einstellbar über den Naphtoesäureanteil (gegenüber dem Vergleichsharzsystem-1 steigt die Brechzahl von > 1,477 auf > 1,535)

### Beispiel 4 Grundharzsynthese 3

Gleichzeitige Umsetzung mit Verbindung (1) und Verbindung (2) im Unterschuss Methacrylsäure (das Produkt entspricht dem in Anspruch 11 beanspruchten Kieselsäurepolykondensat; auf der Ebene der Silane entsteht eine Mischung wie in Anspruch 6 beansprucht.

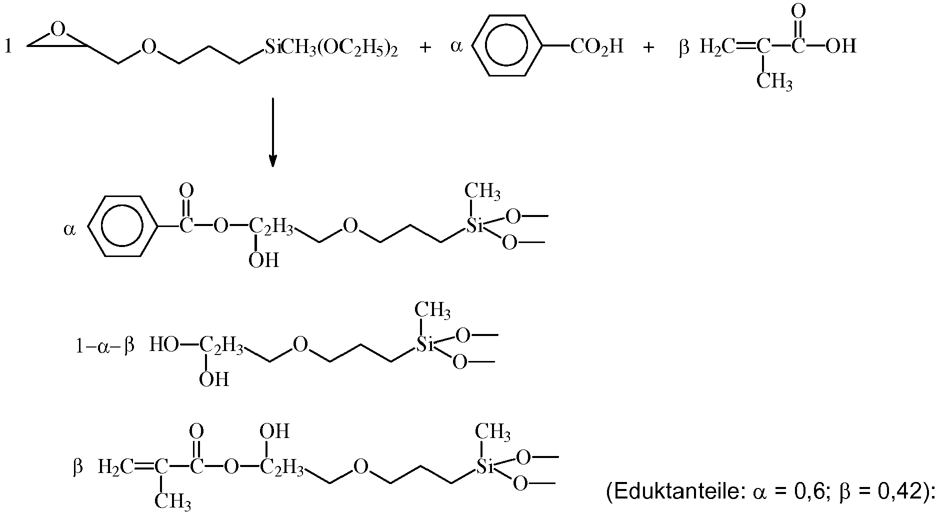

Zur Vorlage von 50,2 g (0,202 mol) 3-Glycidyloxypropylmethyldiethoxysilan werden 0,42 g Triphenyl-phosphin als Kat., 0,04 g BHT (2,6-Di-tert.-butyl-4-methylphenol), 14,7 g (0,12 mol) Benzoesäure und anschließend 7,23 g (0,084 mol) Methacrylsäure zugegeben und bei ≈ 85 °C gerührt (ca. 24 h). Die Umsetzung kann entsprechend Beispiel 1 verfolgt werden. Nach Zugabe von Essigester (1000 ml/mol Silan) und H₂O zur Hydrolyse mit HCl als Kat. wird bei 30 °C gerührt. Der Verlauf der Hydrolyse wird durch Wassertitration verfolgt. Die Aufarbeitung erfolgt nach mehrtägigem Rühren durch Ausschütteln mit wäßriger NaOH und Wasser und Filtration über hydrophobierte Filter. Es wird zunächst abrotiert und anschließend mit Ölpumpenvakuum abgezogen. Es resultiert ein flüssiges Harz ohne Einsatz von Reaktivverdünneren (Monomeren) mit einer Viskosität von ca. 19 - 26 Pa·s bei 25 °C sowie einer Brechzahl n_{D} ≈ 1,506.

### Beispiel 5 Harzsynthese 3a (Herstellung eines Kieselsäurepolykondensats mit Resten R¹, die Gruppen der Formel (II) mit R³ = -D-{B} tragen

Umsetzung des Produkts aus Beispiel 4 mit Isocyanatoethylmethacrylat (Anmerkung: die Isocyanat-Gruppe reagiert nicht nur mit der Hydroxygruppe der Umsetzungsprodukte aus Beispiel 4, sondern auch mit den vicinalen Hydroxygruppen des unumgesetzten Ausgangsmaterials, soweit dessen cyclische Ethergruppen hydrolytisch geöffnet wurden; es resultiert ein Isomeren-Gemisch)*:*

### (Additionsanteile α = 0,6):

Zur Vorlage von 19,9 g (0,07 mol bez. auf Si) der Reaktionsmischung aus Beispiel 4 und ggf. 0,026 g BHT werden unter trockener Atmosphäre bei 30 °C unter Rühren 6,52 g (0,042 mol) Methacrylsäureisocyanatoethylester zugetropft und bei 30 °C weitergerührt. Die Umsetzung kann über die Abnahme der OCN-Bande mittels IR-Spektrum verfolgt werden. Die für die OCN-Gruppe charakteristische Bande erscheint im IR-Spektrum bei 2272 cm⁻¹. Es resultiert ein flüssiges Harz mit einer Viskosität von ca. 102 - 128 Pa·s bei 25 °C sowie einer Brechzahl n_{D} ≈ 1,504.

### Beispiel 6 Harzsynthese 1a

Herstellung eines Kieselsäurepolykondensats mit Gruppen der Formel (II), in denen ein Teil der Reste R³ = OH ist und ein Teil der Reste -D-{B} bedeutet

### Beispiel 6a

Das Produkt des Beispiels 1 mit α = 1 wurde mit Iscyanatoethylmethacrylat (α = 0,8) nach folgendem Schema umgesetzt:

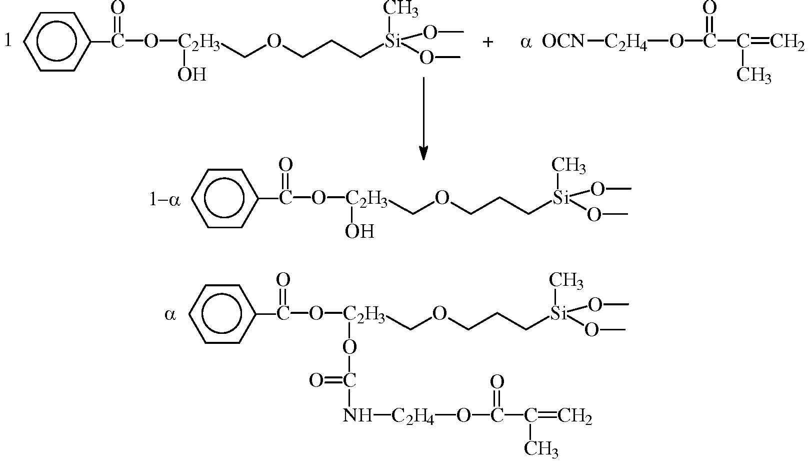

Varianten: Statt mit dem Isocyanatoethylmethacrylat können die (Meth)acrylatgruppen auch durch Addition von (Meth)acrylsäurechlorid bzw. Methacrylsäureanhydrid eingeführt werden.

### Beispiel 6b

Beispiel 6a wurde mit der Änderung wiederholt, dass anstelle des Produkts des Beispiels 1 das Produkt des Beispiels 2 mit α = 1 eingesetzt wurde.

### Beispiel 7 Harzsynthese 1b

Herstellung eines Kieselsäurepolykondensats mit Gruppen (II), in denen ein Teil der Reste R³ -D-{B} und ein weiterer Teil der Reste R³ -D-(W)ₐ(Ar)_{b} bedeutet)

### Beispiel 7a

Das Produkt des Beispiels 1 mit α = 1 wurde mit Isocyanatoethylmethacrylat (α = 0,8) und Phenylisocyanat (β = 0,2) nach folgendem Schema umgesetzt:

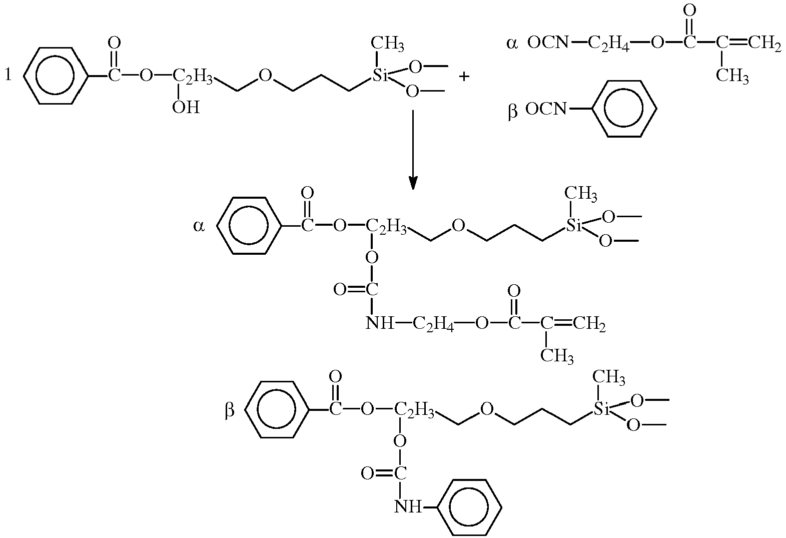

### Beispiel 7b

Beispiel 7a wurde mit der Änderung wiederholt, dass anstelle des Produkts des Beispiels 1 das Produkt des Beispiels 2 mit α = 1 eingesetzt wurde.

### Beispiel 8 Grundharzsynthese 4

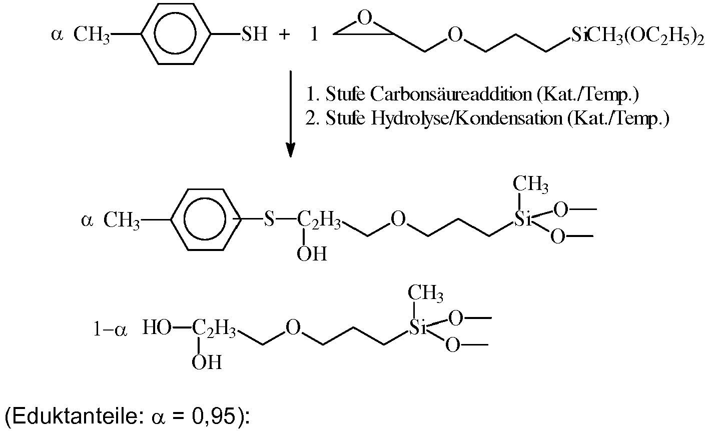

Zur Vorlage von 37,3 g (0,150 mol) 3-Glycidyloxypropylmethyldimethoxysilan werden 0,15 g Triethylamin als Katatalysator und anschließend 17,7 g (0,143 mol) 4-Metylbenzenthiol zugegeben und bei RT gerührt (ca. 30 h). Nach Zugabe von Essigester (1000 ml/mol Silan) und H₂O zur Hydrolyse mit HCl als Kat. wird bei 30 °C gerührt. Der Verlauf der Hydrolyse wird durch Wassertitration verfolgt. Die Aufarbeitung erfolgt nach mehrtägigem Rühren durch Ausschütteln mit wäßriger NaOH und Wasser und Filtration über hydrophobierte Filter. Es wird zunächst abrotiert und anschließend mit Ölpumpenvakuum abgezogen. Es resultiert ein flüssiges Harz ohne Einsatz von Reaktivverdünneren (Monomeren) mit einer Viskosität von ca. 12 Pa·s bei 25 °C sowie einer Brechzahl n_{D} ≈ 1,548.

Weitere Aspekte:
▪ Brechzahl fein einstellbar über den 4-Metylbenzenthiolanteil (gegenüber dem Vergleichsharzsystem-1 steigt die Brechzahl von > 1,477 auf ≈ 1,548)

Variante: Nach der Thioladdition kann ggf. auch ein Teil der verbliebenen Epoxidgruppen (oder vollständig) mit z. B. Methacrylsäure umgesetzt werden (s. Grundharzsynthese 3, Beispiel 4). Die angegebene Reihenfolge ist bevorzugt, da sonst die Gefahr besteht, dass die SH-Gruppe an die Methacrylatgruppe addiert.

### Beispiel 9 Harzsynthese 4a

Herstellung eines Kieselsäurepolykondensats mit Gruppen (II), in denen ein Teil der Reste R³ = OH ist und ein Teil der Reste -D-{B} bedeutet

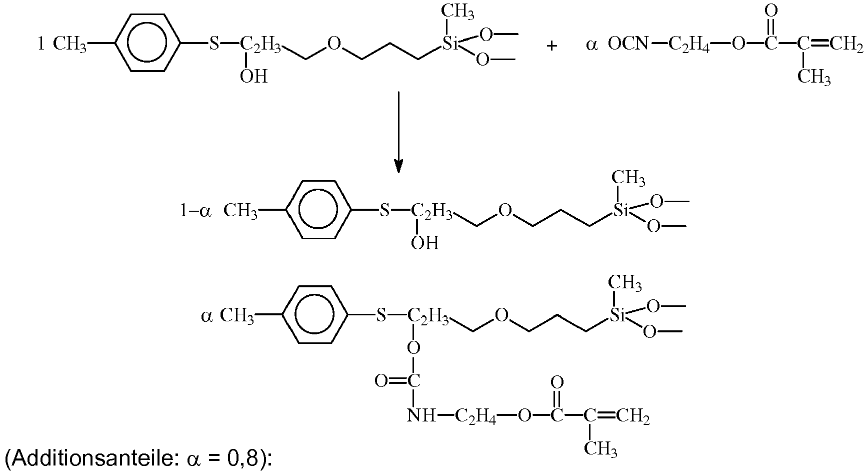

Zur Vorlage von 22,9 g (0,08 mol bez. auf Si) der Reaktionsmischung aus Beispiel 4, 0,1 % BHT und ggf. 0,025 g Dibutylzinnlaurat als Katalysator werden unter trockener Atmosphäre bei 30 °C unter Rühren 9,93 g (0,064 mol) Methacrylsäureisocyanatoethylester zugetropft und bei 30 °C weitergerührt. Die Umsetzung kann über die Abnahme der OCN-Bande mittels IR-Spektrum verfolgt werden. Die für die OCN-Gruppe charakteristische Bande erscheint im IR-Spektrum bei 2272 cm⁻¹. Es resultiert ein flüssiges Harz mit einer Viskosität von ca. 86 Pa·s bei 25 °C sowie einer Brechzahl n_{D} ≈ 1,534.

### Beispiel 10 Grundharzsynthese 5

### Anmerkung: Die Reaktion erfolgt analog zu der des Beispiels 1

### (Eduktanteile: α = 0,95):

Zur Vorlage von 25,1 g (0,10 mol) 3-Glycidyloxypropylmethyldimethoxysilan werden 0,13 g Triphenyl-phosphin als Katalysator und anschließend 18,8 g (0,095 mol) Biphenyl-4-carbonsäure zugegeben und bei ≈ 85 °C gerührt (ca. 1 Tg). Die Umsetzung kann entsprechend Beispiel 1 verfolgt werden. Nach Zugabe von Essigester (1000 ml/mol Silan) und H₂O zur Hydrolyse mit HCl als Kat. wird bei 30 °C gerührt. Der Verlauf der Hydrolyse wird durch Wassertitration verfolgt. Die Aufarbeitung erfolgt nach mehrtägigem Rühren durch Ausschütteln mit wäßriger NaOH und Wasser und Filtration über hydrophobierte Filter. Es wird zunächst abrotiert und anschließend mit Ölpumpenvakuum abgezogen. Es resultiert ein zähflüssiges Harz ohne Einsatz von Reaktiv-verdünneren (Monomeren) mit einer Brechzahl n_{D} ≈ 1,574.

Weitere Aspekte:
▪ Brechzahl fein einstellbar über den Biphenyl-4-carbonsäureanteil (gegenüber dem Vergleichsharzsystem-1 steigt die Brechzahl von > 1,477 auf ≈ 1,574)

### Beispiel 11 Grundharzsynthese 6

### Anmerkung: Die Reaktion erfolgt analog zu der des Beispiels 8

### Beispiel 6 (Eduktanteile: α = 0,95):

Zur Vorlage von 24,9 g (0,10 mol) 3-Glycidyloxypropylmethyldimethoxysilan werden 0,1 g Triethylamin als Katalysator und anschließend 15,2 g (0,095 mol) 2-Naphthalinthiol zugegeben und bei RT - 40 °C gerührt (ca. 1 Tg). Nach Zugabe von Essigester (1000 ml/mol Silan) und H₂O zur Hydrolyse mit HCl als Kat. wird bei 30 °C gerührt. Der Verlauf der Hydrolyse wird durch Wassertitration verfolgt. Die Aufarbeitung erfolgt nach mehrtägigem Rühren durch Ausschütteln mit wäßriger NaOH und Wasser und Filtration über hydrophobierte Filter. Es wird zunächst abrotiert und anschließend mit Ölpumpenvakuum abgezogen. Es resultiert ein zähflüssiges Harz mit einer Viskosität von ca. 630 Pa·s bei 25 °C sowie einer Brechzahl n_{D} ≈ 1,609.

Weitere Aspekte:
▪ Brechzahl fein einstellbar über den 2-Naphthalinthiol-Anteil (gegenüber dem Vergleichsharzsystem-1 steigt die Brechzahl von > 1,477 bis ≈ 1,609)

Anmerkung: Das Harz dieses Beispiels ist in dieser Form noch nicht härtbar. Hierfür müssen über die OH-Gruppen noch polymerisierbare Gruppen (durch z. B, Addition von Methacrylsäureisocyanatoethylester) eingeführt werden.

### B. Mechanische und optische Daten nach Härtung

### Polymerisation/Härtung verschiedener Harzsysteme im Vergleich zum Basisharz ohne die beschriebenen Modifikationen

Das jeweilige Harz aus den Beispielreihen bzw. des Basisharzsystems 1 mit 1% Lucirin TPO wird in eine Stäbchenform (2 x 2 x 25 mm³) gegeben. Die Methacrylatgruppen werden im Rahmen einer photoinduzierten radikalischen Polymerisation umgesetzt, wobei das Harz aushärtet. Mittels 3-Punktbiegeversuch wird nach 1,5 Tagen Lagerung bei 40 °C der E-Modul, die Bruchfestigkeit sowie die Durchbiegung bis zum Bruch der resultierenden Stäbchen bestimmt. Nach entsprechender Herstellung von plättchenförmigen Prüfkörper wird die Brechzahl mittels Refraktometer bestimmt.

**Tab. Mechanische und optische Daten nach Härtung**

| **Harzsystem** | **Bruchfestigkeit [MPa]** | **E-Modul [GPa]** | **Durchbiegung [mm]** | **Brechzahl n_{D}** |
|---|---|---|---|---|
| Vergleichsharzsystem-1 | ≈ 89 | ≈ 1,94 | ≈ 2,4 | 1,504 |
| | | | | |
| Beispiel 4 | n.b. | n.b. | n.b. | ≈ 1,521 |
| Beispiel 5 | ≈ 114 | ≈ 2,45 | ≈ 3,1 | ≈ 1,521 |
| Beispiel 9 | ≈ 70 | ≈ 1,56 | ≈ 3,3 | ≈ 1,551 |

▪ Die Brechzahlen der gehärteten Harze aus den Beispielen 4 und 5 sind gut an den kostengünstigen dentalen Füllstofftyp GM27884 (Schottdentalglas mit n_{D} ≈ 1,53) angepasst womit hochtranzluzente dentale Komposite möglich sind. Hingegen erfordert Vergleichsharzsystem-1 einen sehr teuren dentalen Füllstofftyp GO18-307 (Schottdentalglas mit n_{D} ≈ 1,50) zur Realisierung einer hoher Komposittransluzenz.
▪ Das gehärtete Harzsystem des Beispiels 5 zeigt neben der beschriebenen Brechzahlanpassung zusätzlich signifikant bessere mechanische Daten im Vergleich zum gehärteten Vergleichsharzsystem-1.
▪ Das gehärtete Harzsystem des Beispiels 9 zeigt im Vergleich zum gehärteten Vergleichsharzsystem-1 eine signifikant erhöhte Brechzahl bei guter Flexibilität (s. Durchbiegung).
▪ Weiterhin ist dies ist möglich ohne den Einsatz von dentalen Monomeren (s. zunehmende Allergiediskussion im Dentalbereich).

### Brechzahlen der Harzsysteme

Die bisher bestimmten Harzbrechzahlen liegen im Bereich von 1,504 - 1,609. Die Harzbrechzahl ist somit in einem sehr weiten Bereich einstellbar. Dies gilt selbstverständlich auch für die Brechzahl der gehärteten Systeme (durchschnittlich jeweils um ≈ 0,02 höher).

## Patentansprüche

1. Silan der Formel (I)
R¹ₘR²ₙSiX₄₋ₘ₋ₙ, (I)
worin
R¹ ein über Kohlenstoff am Siliciumatom gebundener kohlenwasserstoffhaltiger, verzweigter oder unverzweigter Rest ist, der an mindestens einem seiner Kohlenstoffatome mit einer Gruppe der Formel (II)
(Ar)_{b}(W)ₐ-A-Y(R³)- (II)
sowie optional mit einem oder mehreren weiteren Substituenten substituiert ist,
worin Ar einen aromatischen Rest bedeutet, der mindestens eine Aryl- und/oder Heteroarylgruppe trägt oder daraus besteht,
W ein substituierter oder unsubstituierter Kohlenwasserstoffrest ist, dessen Kette unterbrochen sein kann durch -S-, -O-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
A entweder zweibindig ist und dann die Bedeutung -O-, -C(O)O-, -S-, -NR⁴- oder -P(O)ₑ(R⁴)_{c}(Z)_{d}- mit Z = OR⁴, c = 0 oder 1, d = 0 oder 1, (c+d) = 1 und e = 0 oder 1 hat oder dreibindig ist und -N= oder -P(O)ₑ= bedeutet,
R⁴ = ein beliebiger kohlenwasserstoffhaltiger Rest ist und im Rest -NR⁴ darüber hinaus außerdem Wasserstoff bedeuten kann,
Y eine aus der Ringöffnung einer reaktiven cyclischen Ethergruppe hervorgegangene Alkylengruppe darstellt, die neben dem in der Formel (II) angegebenen Rest R³ mindestens einen weitere Substituenten tragen kann,
R³ entweder OH ist oder -D-{B} oder -D-(W)ₐ(Ar)_{b} bedeutet, wobei D ausgewählt ist unter einer über Kohlenstoff an {B} oder an W oder, im Falle von a = 0, an Ar gebundenen Carboxylgruppe (Estergruppe), Ethergruppe, Urethangruppe und mit Hydroxy substituierten Ethylenoxygruppe, und {B} ein organisch polymerisierbarer Rest ist, der mindestens eine organisch polymerisierbare C=C-Doppelbindung trägt, und W, Ar, a und b wie für Formel (II) definiert sind,
wobei Ar an W gebunden sein muss, sofern a ≠ o ist,
a 0 oder 1 ist und b 1 oder, im Falle a = 1 und/oder für den Fall, dass A dreibindig ist, eine ganze Zahl größer 1 sein kann,
R² ein über Kohlenstoff an das Silicium gebundener kohlenwasserstoffhaltiger Rest ist,
X eine unter hydrolytischen Bedingungen von Silicium abhydrolysierbare Gruppe oder OH ist,
m 1, 2 oder 3 ist, und
n 0 oder 1 oder 2 ist, mit der Maßgabe, dass m + n 1, 2 oder 3 sind.

2. Silan der Formel (I) nach Anspruch 1,worin der Rest R¹ weiterhin an mindestens einem Kohlenstoffatom mit einer Gruppe substituiert ist, die eine reaktive cyclische Ethergruppe oder zwei vicinale Hydroxygruppen aufweist.

3. Mischung von zwei oder drei Silanen, wobei jedes dieser Silane ausgewählt ist aus einer anderen der folgenden drei Gruppen von Silanen:
(a) Silanen der Formel (I) nach Anspruch 1, worin R³ OH ist,
(b) Silanen der Formel (I) nach Anspruch 1, worin R³ -D-{B} bedeutet, und
(c) Silanen der Formel (I) nach Anspruch 1, worin R³ -D-(W)ₐ(Ar)_{b} bedeutet.

4. Mischung eines Silans der Formel (I) nach Anspruch 1 oder eines Silangemischs nach Anspruch 3 mit einem Silan der Formel (III)
R¹'ₘR²ₙSiX₄₋ₘ₋ₙ, (III),
worin R², X, n und m wie für Formel (I) definiert sind und R¹' ein über Kohlenstoff am Siliciumatom gebundener kohlenwasserstoffhaltiger, verzweigter oder unverzweigter Rest ist, der an mindestens einem seiner Kohlenstoffatome eine reaktive, cyclische Ethergruppe oder zwei an zwei vicinalen Kohlenstoffatomen befindliche Hydroxygruppen sowie - in beiden Fällen - ggf. weitere Substituenten trägt.

5. Mischung eines Silans der Formel (I) wie in Anspruch 1 beansprucht mit einem Silan (IV) der Formel
R¹*ₘR²ₙSiX₄₋ₘ₋ₙ, (IV)
worin R^{1*} ein über Kohlenstoff am Siliciumatom gebundener kohlenwasserstoffhaltiger, verzweigter oder unverzweigter Rest ist, der am mindestens einem seiner Kohlenstoffatome mit einer Gruppe {B}-A"-Y(OH) substituiert ist, worin {B} und Y wie für die Formel (II) in Anspruch 1 definiert sind und A" ausgewählt ist unter C(O)O und O, sowie optional mit einem Silan der Formel (III) wie in Anspruch 4 definiert.

6. Silan nach Anspruch 1, **dadurch gekennzeichnet, dass** der Rest R¹ weiterhin mit einer Gruppe der Formel (V)
{B}-A"-Y(OH) (V)
substituiert ist, worin {B} und Y wie für die Formel (II) in Anspruch 1 definiert sind und A" ausgewählt ist unter C(O)O und O.

7. Organisch modifiziertes Kieselsäure(hetero)polykondensat, enthaltend über Kohlenstoff am Siliciumatom gebundene kohlenwasserstoffhaltige, verzweigte oder unverzweigte Reste R¹, die an mindestens einem Kohlenstoffatom mit einer Gruppe der Formel
(Ar)_{b}(W)ₐ-A-Y(R³)- (II)
sowie optional mit weiteren Substituenten substituiert sind,
worin Ar einen aromatischen Rest bedeutet, der mindestens eine Aryl- und/oder Heteroarylgruppe aufweist oder daraus besteht,
W ein substituierter oder unsubstituierter Kohlenwasserstoffrest ist, dessen Kette unterbrochen sein kann durch -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
A entweder zweibindig ist und dann die Bedeutung -O-, -C(O)O-, -S-, -NR⁴- oder -P(O)ₑ(R⁴)_{c}(Z)_{d}- mit Z = OR⁴, c = 0 oder 1, d = 0 oder 1, (c+d) = 1 und e = 0 oder 1hat oder dreibindig ist und -N= oder -P(O)ₑ= bedeutet,
R⁴ = ein beliebiger kohlenwasserstoffhaltiger Rest ist und im Rest -NR⁴ darüber hinaus außerdem Wasserstoff bedeuten kann,
Y eine aus der Ringöffnung einer reaktiven cyclischen Ethergruppe hervorgegangene Alkylengruppe darstellt, die neben dem in der Formel (I) angegebenen Rest R³ mindestens einen weiteren Substituenten tragen kann,
R³ entweder OH ist oder -D-{B} oder -D-(W)ₐ(Ar)_{b} bedeutet, wobei D ausgewählt ist unter einer über Kohlenstoff an {B} gebundenen Carboxylgruppe (Estergruppe), Ethergruppe, Urethangruppe und mit Hydroxy substituierten Ethylenoxygruppe, und {B} ein organisch polymerisierbarer Rest ist, der mindestens eine C=C-Doppelbindung trägt, und W, Ar, a und b wie für Formel (II) definiert sind,
wobei Ar an W gebunden sein muss, sofern a ≠ 0 ist,
a 0 oder 1 ist und b 1 oder, im Falle a = 1 und/oder für den Fall, dass A dreibindig ist, eine ganze Zahl größer 1 sein kann.

8. Organisch modifiziertes Kieselsäure(hetero)polykondensat nach Anspruch 7 mit mindestens zwei oder drei verschiedenen Gruppen der Formel (II), worin jede der verschiedenen Gruppen ausgewählt ist aus einer anderen der folgenden drei Gruppen der Formel (II):
(a) Gruppen, in denen R³ OH ist,
(b) Gruppen, in denen R³ -D-{B} bedeutet, und
(c) Gruppen, in denen R³ -D-(W)ₐ(Ar)_{b} bedeutet.

9. Organisch modifiziertes Kieselsäure(hetero)polykondensat nach Anspruch 7 oder 8, worin ein Teil oder alle Rest R¹ weiterhin mit einer Gruppe der Formel (V)
{B}-A"-Y(OH)- (V)
substituiert ist, worin {B} und Y wie für die Formel (II) in Anspruch 7 definiert sind und A" ausgewählt ist unter -C(O)O- und -O-.

10. Organisch vernetztes Kieselsäure(hetero)polykondensat nach einem der Ansprüche 7 und 8, umfassend Gruppen der Formel (II), in denen R³ -D-{B} bedeutet, oder nach einem der Ansprüche 11 und 12, worin ein Teil oder alle Reste {B} über eine Polymerisation miteinander vernetzt sind.

11. Komposit, umfassend ein Kieselsäure(hetero)polykondensat nach einem der Ansprüche 7 bis 10 und einen oder mehrere Füllstoffe.

12. Verwendung eines Silans nach einem der Ansprüche 1 bis 6, eines Kieselsäure(hetero)polykondensats nach einem der Ansprüche 7 bis 9, eines organisch vernetzten Kieselsäure(hetero)polykondensats nach Anspruch 10 oder eines Komposits nach Anspruch 11 für dentale Zwecke.

13. Verfahren zum Herstellen eines Silans mit der Formel (I) wie in Anspruch 1 beansprucht, oder eines Kieselsäurepolykondensats wie in Anspruch 7 beansprucht, worin R³ OH bedeutet, **dadurch gekennzeichnet, dass** ein Silan der Formel (III)
R¹'ₘR²ₙSiX₄₋ₘ₋ₙ, (III),
worin R², X, n und m wie für das Silan der Formel (I) definiert sind und R¹' ein über Kohlenstoff am Siliciumatom gebundener kohlenwasserstoffhaltiger, verzweigter oder unverzweigter Rest ist, der an mindestens einem seiner Kohlenstoffatome eine reaktive, cyclische Ethergruppe sowie ggf. weitere Substituenten trägt, mit einer Verbindung der Formel (1)
(Ar)_{b}(W)ₐ-Q (1)
umgesetzt wird, worin Ar, W, a und b wie für den Substituenten der Formel (II) definiert sind und Q ausgewählt ist unter -OH, -C(O)OH, -SH, -NHR⁴, -PH(O)ₑ(R⁴)_{c}(Z)_{d} mit Z = OR⁴, c = 0 oder 1, d = 0 oder 1 und (c+d) = 1, HN= oder HP(O)=, wobei Z und R⁴ wie für Formel (II) in Anspruch 1 definiert sind, wobei das stöchiometrische Verhältnis der Komponenten so gewählt wird, dass die Anzahl der Reste Q mindestens der Anzahl der reaktiven cyclischen Ethergruppen entspricht oder dass die Anzahl der Reste Q geringer ist als die Anzahl der cyclischen Ethergruppen, wobei zur Herstellung des Kieselsäurepolykondensats vor, während oder nach der Umsetzung mit Verbindung (1) eine hydrolytische Kondensationsreaktion durchgeführt wird.

14. Verfahren zum Herstellen eines Silans mit der Formel (I) wie in Anspruch 1 beansprucht oder eines Kieselsäurepolykondensats wie in Anspruch 7 beansprucht, worin R³ -D-{B} bedeutet, **dadurch gekennzeichnet, dass** ein Silan der Formel (I), worin R³ OH ist, umgesetzt wird mit einer Verbindung der Formel (3)
{B}-V (3),
worin {B} die Bedeutung hat wie in Anspruch 1 angegeben und V ausgewählt ist unter -NCO, OH, einer unsubstituierten oder mit Alkyl substituierten Epoxygruppe und dem Rest COA', mit A' = Hydroxy, einem Halogenid oder -OC(O)R⁵ mit R⁵ gleich unsubstituiertem oder substituiertem Kohlenwasserstoff.

15. Verfahren zum Herstellen eines Silans mit der Formel (I) wie in Anspruch 1 beansprucht oder eines Kieselsäurepolykondensats wie in Anspruch 7 beansprucht, worin R³ D-(W)ₐ(Ar)_{b} bedeutet, **dadurch gekennzeichnet, dass** ein Silan der Formel (I), worin R³OH ist, umgesetzt wird mit einer Verbindung der Formel (4)
(Ar)_{b}(W)ₐV, (4)
worin Ar, W, a und b die in Anspruch 1 angegebene Bedeutung besitzen und V ausgewählt ist unter NCO, OH, einer gegebenenfalls alkylsubstituierten Epoxidgruppe und dem Rest COA' mit A' = Hydroxy, einem Halogenid oder -OC(O)R⁵ mit R⁵ gleich unsubstituiertem oder substituiertem Kohlenwasserstoff, oder worin V eine cyclische Anhydridgruppe ist, die über zwei Kohlenstoffatome an W gebunden ist, sofern a = 1 ist, oder worin V ein Anhydrid ist, dessen Kohlenstoffatome jeweils an einen Rest Ar gebunden sind, sofern a = 0 und b = 2 ist, wobei zur Herstellung des Kieselsäurepolykondensats vor, während oder nach der Umsetzung mit Verbindung (4) eine hydrolytische Kondensationsreaktion durchgeführt wird.

## Claims

1. A silane of the formula (I)
R¹ₘR²ₙSiX₄₋ₘ₋ₙ, (I)
wherein
R¹ is a hydrocarbon-containing, branched or unbranched radical bonded to the silicon atom via carbon, which radical is substituted on at least one of its carbon atoms by a group of the formula (II)
(Ar)_{b}(W)ₐ-A-Y(R³)- (II)
and optionally substituted with one or more further substituents,
wherein Ar represents an aromatic radical having or consisting of at least one aryl and/or heteroaryl group,
W is a substituted or unsubstituted hydrocarbon radical whose chain may be interrupted by - S-, -O-, -NR⁴-, -C(O)O-, -NHC(O)-, -C(O)NH-, -NHC(O)NH-, -NHC(O)O-, -C(O)NHC(O)-, - NHC(O)NH-, -S(O)-, -C(S)O-, -C(S)NH-, -NHC(S)NH-, -NHC(S)O-,
A is either divalent and then has the meaning -O-, -C(O)O-, -S-, -NR⁴- or -P(O)ₑ(R⁴)_{c}(Z)_{d}-, wherein Z = OR⁴, c = 0 or 1, d = 0 or 1, (c+d) = 1 and e = 0 or 1, or has three bonds and denotes -N= or -P(O)ₑ=,
R⁴ = any hydrocarbon-containing radical and, in addition, may be hydrogen in the -NR⁴ radical,
Y represents an alkylene group resulting from the ring opening of a reactive cyclic ether group, which may carry at least one further substituent in addition to the radical R³ indicated in the formula (II),
R³ is either OH or -D-{B} or -D-(W)ₐ(Ar)_{b}, wherein D is selected from a carboxyl group (ester group), ether group, urethane group and hydroxy-substituted ethyleneoxy group bonded via carbon to {B} or to W or, in the case of a = 0, to Ar, and {B} is an organically polymerizable radical carrying at least one organically polymerizable C=C double bond, and W, Ar, a and b are as defined for formula (II),
wherein Ar must be bound to W if a is ≠ 0,
a is 0 or 1 and b can be 1 or, in the case of a = 1 and/or in the case where A has three bonds, an integer greater than 1,
R² is a hydrocarbon-containing radical bonded to the silicon via carbon,
X is a group hydrolyzable from silicon under hydrolytic conditions or OH,
m is 1, 2 or 3, and
n is 0 or 1 or 2, provided that m + n is 1, 2 or 3.

2. The silane of formula (I) according to claim 1, wherein R¹ is further substituted on at least one carbon atom with a group having one reactive cyclic ether group or two vicinal hydroxy groups.

3. A mixture of two or three silanes, each of said silanes being selected from a different one of the following three groups of silanes:
(a) silanes of formula (I) according to claim 1 wherein R³ is OH,
(b) silanes of formula (I) according to claim 1 wherein R^{3 is}-D-{B}, and
(c) silanes of formula (I) according to claim 1 wherein R³ is -D-(W)ₐ(Ar)_{b}.

4. A mixture of a silane of formula (I) according to claim 1 or of a silane mixture according to claim 3 with a silane of the formula (III)
R¹'ₘR²ₙSiX₄₋ₘ₋ₙ, (III),
wherein R², X, n and m are as defined for formula (I) and R¹' is a hydrocarbon-containing, branched or unbranched radical to the silicon atom bonded via carbon, which radical carries on at least one of its carbon atoms a reactive cyclic ether group or two hydroxyl groups located on two vicinal carbon atoms and - in both cases - optionally further substituents.

5. A mixture of a silane of formula (I) as claimed in claim 1 with a silane (IV) of formula
R¹*ₘR²ₙSiX_{4-m-n'} (IV)
wherein R^{1*} is a hydrocarbon-containing, branched or unbranched radical bonded to the silicon atom via carbon and substituted on at least one of its carbon atoms with a group {B}-A"-Y(OH) wherein {B} and Y are as defined for formula (II) claim 1 and A" is selected from C(O)O and O, and optionally with a silane of formula (III) as defined claim 4.

6. The silane according to claim 1, **characterized in that** the radical R¹ is further substituted with a group of formula (V)
{B}-A"-Y(OH) (V)
wherein {B} and Y are as defined for formula (II) in claim 1 and A" is selected from C(O)O and O.

7. An organically modified silicic acid (hetero)polycondensate containing hydrocarbon-containing, branched or unbranched radicals R¹ bonded to the silicon atom via carbon and having at least one carbon atom with a group of the formula
(Ar)_{b}(W)ₐ-A-Y(R³)- (II)
and optionally substituted with further substituents,
wherein Ar represents an aromatic radical having or consisting of at least one aryl and/or heteroaryl group,
W is a substituted or unsubstituted hydrocarbon radical whose chain may be interrupted by -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
A is either divalent and then has the meaning -O-, -C(O)O-, -S-, -NR⁴- or -P(O)ₑ(R⁴)_{c}(Z)_{d}-where Z = OR⁴, c = 0 or 1, d = 0 or 1, (c+d) = 1 and e = 0 or 1, or has three bonds and denotes -N= or -P(O)ₑ=,
R⁴ = any hydrocarbon-containing radical and, in addition, may be hydrogen in the -NR4 radical,
Y represents an alkylene group resulting from the ring opening of a reactive cyclic ether group, which may carry at least one further substituent in addition to the radical R³ indicated in the formula (I),
R³ is either OH or -D-{B} or -D-(W)ₐ(Ar)_{b}, wherein D is selected from a carboxyl group (ester group), ether group, urethane group and hydroxy-substituted ethyleneoxy group bonded to {B} via carbon, and {B} is an organically polymerizable radical bearing at least one C=C double bond, and W, Ar, a and b are as defined for formula (II),
where Ar must be bound to W if a ≠ is 0,
a is 0 or 1 and b can be 1 or, in the case of a = 1 and/or in the case where A has bonds, an integer greater than 1.

8. The organically modified silica (hetero)polycondensate of claim 7 having at least two or three different groups of formula (II) wherein each of the different groups is selected from one of the following three groups of formula (II):
(a) groups wherein R³ is OH,
(b) groups wherein R^{3 is} -D-{B}, and
(c) groups wherein R³ is -D-(W)ₐ(Ar)_{b}.

9. The organically modified silicic acid (hetero)polycondensate according to claim 7 or 8 wherein a part or all of R¹ is further substituted with a group of formula (V)
{B}-A"-Y(OH)- (V)
wherein {B} and Y are as defined for formula (II) in claim 7 and A" is selected from -C(O)O- and -O-.

10. The organically crosslinked silicic acid (hetero)polycondensate according to any one of claims 7 and 8 comprising groups of formula (II) in which R³ is -D-{B}, or according to any one of claims 11 and 12, wherein some or all of {B} are crosslinked by polymerization.

11. A composite comprising a silica (hetero)polycondensate according to any one of claims 7 to 10 and one or more fillers.

12. Use for dental purposes of a silane according to any of claims 1 to 6, a silicic acid (hetero)polycondensate according to any of claims 7 to 9, an organically crosslinked silicic acid (hetero)polycondensate according to claim 10 or a composite according to claim 11.

13. A process for preparing a silane having the formula (I) as claimed in claim 1, or a silica polycondensate as claimed in claim 7, wherein R³ is OH, **characterized in that** a silane having the formula (III)
R¹'ₘR²ₙSiX₄₋ₘ₋ₙ, (III),
wherein R², X, n and m are as defined for the silane of the formula (I) and R¹' is a hydrocarbon-containing, branched or unbranched radical bonded to the silicon atom via carbon, which radical carries on at least one of its carbon atoms a reactive cyclic ether group and optionally further substituents, with a compound of the formula (1)
(Ar)_{b}(W)ₐ-Q (1)
wherein Ar, W, a and b are as defined for the substituent of formula (II) and Q is selected from -OH, -C(O)OH, -SH, -NHR⁴, -PH(O)ₑ(R⁴)_{c}(Z)_{d} with Z = OR⁴, c = 0 or 1, d = 0 or 1 and (c+d) = 1, HN= or HP(O)=, wherein Z and R⁴ are as defined for formula (II) in claim 1, wherein the stoichiometric ratio of the components is selected such that the number of radicals Q corresponds at least to the number of reactive cyclic ether groups or that the number of radicals Q is less than the number of cyclic ether groups, wherein a hydrolytic condensation reaction is carried out to prepare the silica polycondensate before, during or after the reaction with compound (1).

14. A process for preparing a silane having the formula (I) as claimed in claim 1 or a silicic acid polycondensate as claimed in claim 7, wherein R³ is -D-{B}, **characterised in that** a silane having the formula (I), wherein R³ is OH, is reacted with a compound having the formula (3)
{B}-V (3),
wherein {B} is as defined in claim 1 and V is selected from -NCO, OH, an unsubstituted or alkyl substituted epoxy group and the radical COA', wherein A' = hydroxy, a halide or -OC(O)R⁵ where R⁵ is an unsubstituted or substituted hydrocarbon.

15. A process for preparing a silane having the formula (I) as claimed in claim 1 or a silicic acid polycondensate as claimed in claim 7 wherein R³ is D-(W)ₐ(Ar)_{b} **characterised in that** a silane having the formula (I), wherein R³ is OH, is reacted with a compound having the formula (4)
(Ar)_{b}(W)ₐV, (4)
wherein Ar, W, a and b are as defined above and V is selected from NCO, OH, an optionally alkyl-substituted epoxide group and the residue COA' wherein A' = hydroxy, a halide or -OC(O)R⁵ where R⁵ is unsubstituted or substituted hydrocarbon, or wherein V is a cyclic anhydride group, which is bonded to W via two carbon atoms when a = 1, or wherein V is an anhydride whose carbon atoms are each bonded to a radical Ar when a = 0 and b = 2, wherein a hydrolytic condensation reaction is carried out to produce the silica polycondensate before, during or after the reaction with compound (4).

## Revendications

1. Silane de la formule (I)
R¹ₘR²ₙSiX₄₋ₘ₋ₙ, (I)
dans laquelle
R¹ est un radical ramifié ou non ramifié, contenant un hydrocarbure, lié à l'atome de silicium par du carbone, qui est substitué à au moins un de ses atomes de carbone avec un groupe de la formule (II)
(Ar)_{b}(W)ₐ-A-Y(R³)- (II)
ainsi que de façon optionnelle avec un ou plusieurs autres substituants,
dans laquelle Ar signifie un radical aromatique, qui porte au moins un groupe aryle et/ou hétéroaryle ou en est constitué,
W est un radical hydrocarboné substitué ou non substitué, dont la chaîne peut être interrompue par -S-, -O-, -NR⁴-, -C(O)O-, -NHC(O)-, -C(O)NH-, -NHC(O)O-,-C(O)NHC(O)-, -NHC(O)NH-, -S(O)-, -C(S)O-, -C(S)NH-,-NHC(S)-, -NHC(S)O-,
A est soit bivalent et a alors la signification -O-,-C(O)O-, -S-, -NR⁴- ou -P(O)ₑ(R⁴)_{c}(Z)_{d}- avec Z=OR⁴, c=0 ou 1, d=0 ou 1, (c+d)=1 et e=0 ou 1 soit trivalent et signifie -N= ou -P(O)ₑ=,
R⁴ est un radical quelconque contenant un hydrocarbure et dans le radical -NR⁴ peut par ailleurs en outre signifier un hydrogène,
Y représente un groupe alkylène issu de l'ouverture d'anneau d'un groupe éther cyclique réactif, qui peut porter au moins un autre substituant en plus du radical R³ indiqué dans la formule (II),
R³ soit est OH soit signifie -D-{B} ou -D(W)ₐ(Ar)_{b}, dans laquelle D est sélectionné parmi un groupe carboxyle (groupe ester), un groupe éther, un groupe uréthane et un groupe éthylènoxy substitué avec hydroxy, lié par du carbone à {B} ou à W ou, dans le cas où a=0, à Ar, et {B} est un radical organiquement polymérisable, qui porte au moins une liaison double C=C organiquement polymérisable, et W, Ar, a et b sont définis comme pour la formule (II),
dans lequel Ar doit être lié à W dans la mesure où a≠0, a est 0 ou 1 et b peut être 1 ou, dans le cas où a=1 et/ou dans le cas où A est trivalent, un nombre entier plus grand que 1,
R² est un radical contenant un hydrocarbure lié au silicium par du carbone,
X est un groupe hydrolysable dans des conditions hydrolytiques du silicium ou OH,
m est 1, 2 ou 3, et
n est 0 ou 1 ou 2, à la condition que m+n soient 1, 2 ou 3.

2. Silane de la formule (I) selon la revendication 1, dans laquelle le radical R¹ est en outre substitué à au moins un atome de carbone avec un groupe, qui présente un groupe éther cyclique réactif ou deux groupes hydroxy vicinaux.

3. Mélange de deux ou trois silanes, dans lequel chacun de ces silanes est sélectionné parmi un autre des trois groupes de silanes suivants:
(a) silanes de la formule (I) selon la revendication 1, dans laquelle R³ est OH,
(b) silanes de la formule (I) selon la revendication 1, dans laquelle R³ signifie -D-{B}, et
(c) silanes de la formule (I) selon la revendication 1, dans laquelle R³ signifie -D-(W)ₐ(Ar)_{b}.

4. Mélange d'un silane de la formule (I) selon la revendication 1 ou d'un mélange de silanes selon la revendication 3 avec un silane de la formule (III)
R¹'ₘR²ₙSiX₄₋ₘ₋ₙ (III)
dans laquelle R², X, n et m sont définis comme pour la formule (I) et R¹' est un radical ramifié ou non ramifié, contenant un hydrocarbure, lié à l'atome de silicium par du carbone, qui porte sur au moins un de ses atomes de carbone un groupe éther cyclique réactif ou deux groupes hydroxy se trouvant sur deux atomes de carbone vicinaux ainsi que - dans les deux cas - le cas échéant d'autres substituants.

5. Mélange d'un silane de la formule (I) comme revendiqué dans la revendication 1 avec un silane (IV) de la formule
R¹'ₘR²ₙSiX₄₋ₘ₋ₙ (IV)
dans laquelle R¹' est un radical ramifié ou non ramifié contenant un hydrocarbure, lié à l'atome de silicium par du carbone, qui est substitué à au moins de ses atomes de carbone avec un groupe {B}-A"-Y(OH), dans laquelle {B} et Y sont définis comme pour la formule (II) dans la revendication 1 et A" est sélectionné parmi C(O)O et O, ainsi que de façon optionnelle avec un silane de la formule (III) comme défini dans la revendication 4.

6. Silane selon la revendication 1, **caractérisé en ce que** le radical R¹ est en outre substitué avec un groupe de la formule (V)
{B}-A"-Y(OH) (V)
dans laquelle {B} et Y sont définis comme pour la formule (II) dans la revendication 1 et A" est sélectionné parmi C(O)O et O.

7. (Hétéro)polycondensat d'acide silicique organiquement modifié, contenant des radicaux R¹ ramifiés ou non ramifiés, contenant un hydrocarbure, liés à l'atome de silicium par du carbone, qui sont substitués à au moins un atome de carbone avec un groupe de la formule
(Ar)_{b}(W)ₐ-A-Y(R³) (II)
ainsi que de façon optionnelle avec d'autres substituants,
dans laquelle Ar signifie un radical aromatique, qui porte au moins un groupe aryle et/ou hétéroaryle ou en est constitué,
W est un radical hydrocarboné substitué ou non substitué, dont la chaîne peut être interrompue par -S-, -O-, -NH-, -NR⁴-, -C(O)O-, -NHC(O)-, -C(O)NH-,-NHC(O)O-, -C(O)NHC(O)-, -NHC(O)NH-, -S(O)-, -C(S)O-,-C(S)NH-, -NHC(S)-, -NHC(S)O-,
A est soit bivalent et a alors la signification -O-,-C(O)O-, -S-, -NR⁴- ou -P(O)ₑ(R⁴)_{c}(Z)_{d}- avec Z=OR⁴, c=0 ou 1, d=0 ou 1, (c+d)=1 et e=0 ou 1 soit trivalent et signifie -N= ou -P(O)ₑ=,
R⁴ est un radical quelconque contenant un hydrocarbure et dans le radical -NR⁴ peut par ailleurs en outre signifier un hydrogène,
Y représente un groupe alkylène issu de l'ouverture d'anneau d'un groupe éther cyclique réactif, qui peut porter au moins un autre substituant en plus du radical R³ indiqué dans la formule (I),
R³ soit est OH soit signifie -D-{B} ou -D(W)ₐ(Ar)_{b}, dans laquelle D est sélectionné parmi un groupe carboxyle (groupe ester), un groupe éther, un groupe uréthane et un groupe éthylènoxy substitué avec hydroxy, lié par du carbone à {B}, et {B} est un radical organiquement polymérisable, qui porte au moins une liaison double C=C, et W, Ar, a et b sont définis comme pour la formule (II),
dans lequel Ar doit être lié à W dans la mesure où a≠0, a est 0 ou 1 et b peut être 1 ou, dans le cas où a=1 et/ou dans le cas où A est trivalent, un nombre entier plus grand que 1.

8. (Hétéro)polycondensat d'acide silicique organiquement modifié selon la revendication 7, avec au moins deux ou trois groupes différents de la formule (II), dans lequel chacun des groupes différents est sélectionné parmi un autre des trois groupes suivants de la formule (II) :
(a) groupes, dans lesquels R³ est OH,
(b) groupes, dans lesquels R³ signifie -D-{B}, et
(c) groupes, dans lesquels R³ signifie -D-(W)ₐ(Ar)_{b}.

9. (Hétéro)polycondensat d'acide silicique organiquement modifié selon la revendication 7 ou 8, dans lequel une partie ou tout le radical R¹ est en outre substitué avec un groupe de la formule (V)
{B}-A"-Y(OH)- (V)
dans laquelle {B} et Y sont définis comme pour la formule (II) dans la revendication 7 et A" est sélectionné parmi -C(O)O- et -O-.

10. (Hétéro)polycondensat d'acide silicique organiquement réticulé selon une des revendications 7 et 8, comprenant des groupes de la formule (II), dans lesquels R³ signifie -D-{B}, ou selon une des revendications 11 et 12, dans lequel une partie ou la totalité des radicaux {B} sont réticulés l'un avec l'autre par une polymérisation.

11. Composite, comprenant un (hétéro)polycondensat d'acide silicique selon l'une quelconque des revendications 7 à 10 et une ou plusieurs matière(s) de charge.

12. Utilisation d'un silane selon l'une quelconque des revendications 1 à 6, d'un (hétéro)polycondensat d'acide silicique selon l'une quelconque des revendications 7 à 9, d'un (hétéro)polycondensat d'acide silicique organiquement réticulé selon la revendication 10 ou d'un composite selon la revendication 11 pour des applications dentaires.

13. Procédé de fabrication d'un silane de la formule (I) comme revendiqué dans la revendication 1, ou d'un polycondensat d'acide silicique comme revendiqué dans la revendication 7, dans laquelle R³ signifie OH, **caractérisé en ce que** l'on fait réagir un silane de la formule (III)
R¹'ₘR²ₙSiX₄₋ₘ₋ₙ (III),
dans laquelle R², X, n et m sont définis comme pour le silane de la formule (I) et R¹' est un radical ramifié ou non ramifié, contenant un hydrocarbure, lié à l'atome de silicium par du carbone, qui porte sur au moins un de ses atomes de carbone un groupe éther cyclique réactif ainsi que le cas échéant d'autres substituants, avec un composé de la formule (1)
(Ar)_{b}(W)ₐ-Q (1),
dans laquelle Ar, W, a et b sont définis comme pour le substituant de la formule (II) et Q est sélectionné parmi -OH, -C(O)OH, -SH, -NHR⁴, -PH(O)ₑ(R⁴)_{c}(Z)_{d} avec Z=OR⁴, c=0 ou 1, d=0 ou 1 et (c+d)= 1, HN= ou HP(O)=, dans lequel Z et R⁴ sont définis comme pour la formule (II) dans la revendication 1, dans lequel on choisit le rapport stoechiométrique des composants de telle manière que le nombre des radicaux Q corresponde au moins au nombre de groupes éther cycliques réactifs ou que le nombre des radicaux Q soit inférieur au nombre des groupes éther cycliques, dans lequel pour la fabrication du polycondensat d'acide silicique on effectue une réaction de condensation hydrolytique avant, pendant ou après la réaction avec le composé (1).

14. Procédé de fabrication d'un silane de la formule (I) comme revendiqué dans la revendication 1, ou d'un polycondensat d'acide silicique comme revendiqué dans la revendication 7, dans laquelle R³ signifie -D-{B}, **caractérisé en ce que** l'on fait réagir un silane de la formule (I), dans laquelle R³ est OH, avec un composé de la formule (3)
{B}-V (3),
dans laquelle {B} a la signification indiquée dans la revendication 1 et V est sélectionné parmi -NCO, OH, un groupe époxy non substitué ou substitué avec alkyle et le radical COA', avec A' = hydroxy, un halogénure ou-OC(O)R⁵ avec R⁵ = hydrocarbure non substitué ou substitué.

15. Procédé de fabrication d'un silane de la formule (I) comme revendiqué dans la revendication 1, ou d'un polycondensat d'acide silicique comme revendiqué dans la revendication 7, dans laquelle R³ signifie D-(W)ₐ(Ar)_{b}, **caractérisé en ce que** l'on fait réagir un silane de la formule (I), dans laquelle R³ est OH, avec un composé de la formule (4)
(Ar)_{b}(W)ₐV (4),
dans laquelle Ar, W, a et b possèdent la signification indiquée dans la revendication 1 et V est sélectionné parmi NCO, OH, un groupe époxyde éventuellement substitué avec alkyle et le radical COA' avec A' = hydroxy, un halogénure ou -OC(O)R⁵ avec R⁵ = hydrocarbure non substitué ou substitué, ou dans laquelle V est un groupe anhydride cyclique, qui est lié à W par deux atomes de carbone, dans la mesure où a=1, ou dans laquelle V est un anhydride, dont les atomes de carbone sont respectivement liés à un radical Ar, dans la mesure où a=0 et b=2, dans lequel pour la fabrication du polycondensat d'acide silicique on effectue une réaction de condensation hydrolytique avant, pendant ou après la réaction avec le composé (4).
